(19) **Europäisches Patentamt / European Patent Office / Office européen des brevets**

(11) **EP 4 583 756 B1**

(12) # EUROPEAN PATENT SPECIFICATION

(45) Date of publication and mention
of the grant of the patent:
**15.04.2026  Bulletin 2026/16**

(21) Application number: **23800841.1**

(22) Date of filing: **03.11.2023**

(51) International Patent Classification (IPC):
*A61B 3/00* *(2006.01)*    *A61B 3/032* *(2006.01)*
*A61B 3/10* *(2006.01)*

(52) Cooperative Patent Classification (CPC):
**A61B 3/0025; A61B 3/0041; A61B 3/032;
A61B 3/1005**

(86) International application number:
**PCT/EP2023/080646**

(87) International publication number:
**WO 2024/094839 (10.05.2024 Gazette 2024/19)**

(54) **COMPUTER-IMPLEMENTED METHODS AND DEVICES FOR DETERMINING REFRACTIVE ERRORS**

COMPUTERIMPLEMENTIERTE VERFAHREN UND VORRICHTUNGEN ZUR BESTIMMUNG VON BRECHUNGSFEHLERN

PROCÉDÉS ET DISPOSITIFS MIS EN UVRE PAR ORDINATEUR POUR DÉTERMINER DES ERREURS DE RÉFRACTION

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **04.11.2022  EP 22205588**

(43) Date of publication of application:
**16.07.2025  Bulletin 2025/29**

(73) Proprietor: **Carl Zeiss Vision International GmbH
73430 Aalen (DE)**

(72) Inventors:
• **OHLENDORF, Arne
  73447 Oberkochen (DE)**

• **HUEBNER, Julia
  73447 Oberkochen (DE)**
• **LEUBE, Alexander
  73447 Oberkochen (DE)**

(74) Representative: **Altmann Stößel Dick
Patentanwälte PartG mbB
Theodor-Heuss-Anlage 2
68165 Mannheim (DE)**

(56) References cited:
**EP-A1- 3 329 836      WO-A1-2021/122640
US-A1- 2017 027 436    US-A1- 2022 313 080**

## Description

The present invention relates to a computer-implemented method and a determining device for determining at least one refractive error of an eye of a person; a computer program; a remote device for generating outcome data for determining at least one refractive error of an eye of a person; a determining device for determining at least one refractive error of an eye of a person; a method for producing a geometrical model of at least one spectacle lens for manufacturing of the at least one spectacle lens; and a method for producing at least one spectacle lens.

Related art

Robert E. Bannon, A Study of Astigmatism at the near point with special reference to astigmatic accommodation, American Journal of optometry and Archives of American academy of optometry, Vol. 23, Number 2, February 1946 describes that the present-day refractionist is giving increasingly more attention to findings obtained when the patient's fixation is at or within arm's length. Studies made in industries, schools and business offices have emphasized the need for more attention to visual problems at the near point. The determination of the amount and axis of astigmatism is about the only test made for distance fixation but neglected at the near point.

Kenneth N. Ogle and J. Theodore Schwartz, Depth of Focus of the Human Eye, Journal of the optical society of America, Vol. 49, Number 3, March 1959 describes that a depth of focus of the human eye is determined by the loss of resolving power (visual acuity) with increase in out-of-focus blurring of the retinal image. An instrument is described which permits accurate measurement of these phenomena, using psychophysical methods with the checkerboard visual-acuity test chart. All test conditions are held constant, binocular vision is maintained, and time of exposure of the test target is limited to 0.2 second. Depth of focus was measured under the two following conditions: (1) increase in the angular size of the test target, and (2) increase and decrease of pupil size as effected by suitable drugs.

Alexander Leube, Caroline Kraft, Arne Ohlendorf and Siegfried Wahl, Self-assessment of refractive errors using a simple optical approach, Clinical and Experimental Optometry, 101:3, 386-391, DOI: 10.1111/cxo.12650, 2018 describes that eighteen participants with a mean age of $34.0 \pm 8.8$-years were enrolled. Adjustable Alvarez lenses were mounted in a rotatable ring holder and two procedures were tested for the self-adjustment: (1) rotation of the lens in three meridians: 0°, 60° and 120° and (2) rotation of the optotypes in the same meridians. Starting from maximum positive power, the participants were required to decrease the power of the Alvarez lens until the optotypes (0.0 logMAR) appeared to be clear the first time. Best-corrected visual acuity (BVA) was measured using a psychophysical staircase procedure. Bland-Altmann analysis was carried out in order to calculate the limits of agreement between the self-refraction method and the standard subjective refraction.

WO 2022/090376 A1 discloses a method, computer program and a device for determining at least one refractive error of at least one eye of a person using at least one mobile communication device. Herein, the method comprising the following steps: a) displaying at least one illumination pattern to at least one eye of a person; b) capturing at least one image picturing at least one reflection of the at least one illumination pattern illuminating at least one portion of the at least one eye of the person; and c) determining a value for at least one refractive error of the at least one eye of the person by processing the at least one image, wherein the at least one mobile communication device comprises a screen which is configured to perform step a) and at least one camera which is configured to perform step b). The method, the computer program and the device allow determining the at least one refractive error of at least one eye of a person using at least one mobile communication device with increased precision, accuracy, and reliability.

US 10 702 143 B2 discloses a method including the following steps: (a) displaying at least one dynamic target image of at least one sign over a display area; (b) receiving subjective feedback from the subject indicating that the subject is positioned at a maximum distance of best acuity (MDBA) from the target image, wherein the MDBA is the maximum distance in which the subject recognizes the sign; (c) measuring one or more parameter associated with distance, during the time the subject has reached the MDBA distance, using at least one sensor; (d) estimating the MDBA by estimating the distance between the eye of the subject and the display area in which the target image is displayed by using the sensor data and (e) calculating the refractive error of the eye according to the estimated MDBA and characteristics of the target image.

EP 3 730 038 A1 discloses a system and method for interactively measuring ocular refractive errors, addition and power of reading glasses without the need of an optical component that would change optical vergence of a target. The system can measure the distance from a user's eyes to either or both border of interval of clear vision, for one orientation or two perpendicular orientations. The measurements together with the user's age can be used to estimate sphero-cylindrical refraction, addition and power of reading glasses. The system can use different sizes, directions and colors of targets which can change with said distance or user interaction.

WO 2021 122 640 A1 discloses a system for determining refraction features of both first and second eyes of a subject. The system comprises a light field display device, a system of localization of the positions of the first and second eyes of the subject, the light field display device being configured to generate a first light field directed selectively toward the first eye, and, respectively, a second light field directed selectively toward the second eye, a control unit adapted to adjust said first light field as a function of at least a first refraction parameter associated with at least a first optical aberration for said first eye and the control unit being adapted to adjust said second light field as a function of at least a second refraction parameter associated with at least a second optical aberration for the second eye.

US 2022 0 313 080 A1 discloses apparatus, systems, and methods for objectively assessing accommodation in an eye. For example, a method for objectively assessing accommodation can comprise displaying, at a far display, a stimulus target for a first duration and displaying, at a near display, the stimulus target for a second duration. The stimulus target displayed at the near display can be projected onto a first beam splitter positioned at an oblique angle with respect to the near display. The stimulus target displayed on the far display can be axially aligned with the stimulus target projected onto the first beam splitter. The method can also comprise obtaining, at a controller, measurements concerning refractive states of the eye during the first duration and the second duration from a refractor device in communication with the controller and determining, using the controller, an accommodative response of the eye based in part on the respective refractive states.

Problem to be solved

It is therefore an objective of the present invention, particularly in view of EP 3 730 038 A1, WO 2021 122 640 A1 and US 2022 0 313 080 A1 to provide a computer-implemented method and a determining device for determining at least one refractive error of an eye of a person; a computer program; a data carrier signal; a field device for generating input data for determining at least one refractive error of an eye of a person; a remote device for generating outcome data for determining at least one refractive error of an eye of a person; a determining device for determining at least one refractive error of an eye of a person; a method for producing a geometrical model of at least one spectacle lens for manufacturing of the at least one spectacle lens; and a method for producing at least one spectacle lens, which at least partially overcome the above-mentioned problems of the state of the art.

It is a particular objective of the present invention to provide a fast, easy, versatile, reliable, and accurate approach for determining at least one refractive error of an eye of a person. It may be particularly an objective of the present invention to provide an individual at least one refractive error of the eye of the person, particularly whereby the at least one refractive error may be determined more exactly. It may further be object of the present invention to provide a method for measuring a refractive error of at least one eye of a person, which does not require the use of refractive correction glasses during the measurement process. It may further be object of the present invention to provide a sensor-based distance measurement in real time having a reduced error, particularly in the distance measurement, particularly by considering corresponding meridians.

Summary of the invention

This problem is solved by a computer-implemented method and a determining device for determining at least one refractive error of an eye of a person according to claims 1 and 9, respectively; a computer program according to claim 7; a data carrier signal not forming part of the invention; a field device for generating input data for determining at least one refractive error of an eye of a person not forming part of the invention; a remote device for generating outcome data for determining at least one refractive error of an eye of a person according to claim 8; a method for producing a geometrical model of at least one spectacle lens for manufacturing of the at least one spectacle lens according to claim 10; and a method for producing at least one spectacle lens according to claim 11.

Preferred embodiments of the invention are listed in the dependent claims.

In a first aspect, the present invention relates to a computer-implemented method for determining at least one refractive error of an eye of a person.

As generally used, the term "computer-implemented method" refers to a method which involves at least one apparatus, specifically a computer, or a plurality of apparatus, particularly connected via a computer network. The plurality of apparatus may be connected, particularly for transmitting data, via a network by using at least one connection interface at any one of the apparatuses of the plurality of apparatus. The computer-implemented method may be implemented as at least one computer program that may be provided on a storage medium carrying the computer program, whereby at least one of the steps of the computer-implemented method, specifically at least one of steps a) or b), are performed by using the at least one computer program. Preferably any one of the steps a) and b) are performed using the at least one computer program. Alternatively, the at least one computer program may be accessible by an apparatus which may be adapted for performing the method via a network, such as via an in-house network, via internet, or via a cloud. With particular regard to the present invention, the present method can, thus, be performed on a programmable apparatus, which is configured for this purpose, such as by providing a computer program, which is configured for such a purpose.

As generally used, the term "determining" or any grammatical variation thereof refers to a process configured for generating at least one representative result. The representative result may be determined in a process, wherein at least one step of the process may be selected from at least one of: a measurement step; an evaluation step; or a displaying step.

For determining the representative result, at least one measurement may be performed. Data generated in this measurement may be evaluated. The representative result may be data retrieved in the evaluation process. The representative result may be displayed. With particular regard to the present invention, the representative result comprises outcome data comprising at least one refractive error of the eye of the person. The outcome data may be displayed on at least one screen.

As generally used, the terms "refraction" or "refractive" refer to a bending of incident light entering the interior of the eye of the person via a pupil, wherein the term "refractive error" refers to an observation that the incident light may, in particular owing to a form of the eye, not be focused appropriately on the retina of the eye, resulting particularly in a blurred vision of the person.

The method may be performed for exactly one eye of the person simultaneously. The method may be repeated for a further eye of the person. The method may further be implemented for being performed for at least one eye of the person or both eyes of the person or any eye of the person simultaneously.

The method for determining at least one refractive error of an eye of a person comprises the following steps, which may be performed in the given order. A different order, however, may also be feasible. Further, two or more of the method steps may be performed simultaneously. Thereby the method steps may at least partly overlap in time. Further, the method steps may be performed once or repeatedly. Thus, one or more or even all of the method steps may be performed once or repeatedly. The method may comprise additional method steps, which are not listed herein.

The method comprises the steps defined in claim 1 including, inter alia, the following steps:

a) generating input data configured to comprise

- at least one distance between the eye of the person and at least one visual stimulus displayed to the eye of the person, wherein the at least one distance between the eye of the person is determined by

  ◦ displaying to the eye of the person at least one visual stimulus on at least one screen while the distance between the eye of the person and the at least one visual stimulus displayed to the eye of the person is changed;
  ◦ recording the at least one at least one distance by using a distance measuring unit at a point in time at which a visual perception of at least one spatial characteristic of the at least one visual stimulus perceived by the person changes;

- a position of an entrance pupil of the eye of the person;

b) generating outcome data configured to comprise

- at least one refractive error of the eye of the person, wherein the at least one refractive error of the eye of the person is determined by evaluating the input data, wherein the position of the entrance pupil of the eye of the person is considered when the at least one refractive error of the eye of the person is determined.

According to step a), input data is generated. As used herein, the term "generating" or any grammatical deviation thereof refers to a process of producing a set or a sequence of data, particularly by taking an initial set of data, specifically measurement data, and applying a mathematical or logical process to it. The term does not necessarily imply that a measurement process occurs. For generating input data, it may be sufficient to provide the data that is comprised by the input data. Thereby, the process may make use of an algorithm or a machine learning model. The produced set of data may be the input data. As used herein, the term "input data" refers to a set or sequence of data that comprises information that are at least partly evaluated by a computer-implemented method, particularly the input data may be required for starting at least one step of the method, specifically the computer-implemented method for determining at least one refractive error of an eye of a person. The input data may be required for generating the outcome data.

The input data according to step a) is configured to comprise

- at least one distance between the eye of the person and at least one visual stimulus displayed to the eye of the person, wherein the at least one distance between the eye of the person is determined by

  o displaying to the eye of the person at least one visual stimulus on at least one screen while the distance between the eye of the person and the at least one visual stimulus displayed to the eye of the person is changed;
  ◦ recording the at least one at least one distance by using a distance measuring unit at a point in time at which a visual perception of at least one spatial characteristic of the at least one visual stimulus perceived by the person changes.

As generally used, the term "distance" refers to the shortest connection between two points, particularly the shortest connection eye of the person and the at least one visual stimulus. With particular regard to the invention the shortest connection may consider the optical path the light impinging on the eye is propagating from the at least one stimulus to the eye. As used herein, the term "changing" or any grammatical variation thereof refers to a process of altering a value of an observable, particularly the value of the distance. By changing the observable, the value attributed to the observable before altering the observable may differ from a value attributed to the observable after altering the observable. With respect to the invention, a first distance before changing the distance may differ from a second distance after changing the distance. The distance may be change continuously and, further, particularly in a monotone manner. The distance may be a far point distance or a near point distance.

As used herein, the term "visual stimulus" refers to a graphical presentation of an item, particularly an item that is known or reasonably to be expected by the person skilled in the art to be considerably suitable for determining at least one refractive error of an eye of a person. The visual stimulus may particularly be suitable if it is perceptible by the person, especially due to a contrast between the visual stimulus and a background that allows the eye of the person to distinguish between the visual stimulus and the background.

As generally used, the term "displaying" or any grammatical deviation thereof refers to a presentation of at least one of an image, an item, a text, or a video, particularly at the at least one visual stimulus, on the at least one screen. As generally used, the term "screen" refers to an electronic visual display device designated for the presentation of at least one of an image, an item, text, or a video transmitted electronically. With particular regard to the present invention, the screen may be configured for displaying the at least one visual stimulus to the eye of a person, particularly in such manner that the at least one first visual stimulus may be perceptible by the person.

As generally used, the term "recording" or any grammatical variation thereof refers to a process of generating data, particularly comprised by the input data. The data may be produced in a measurement process. As further used herein, the term "distance measuring unit" refers to an electronic component suitable for generating data, particularly distance measurement data comprised by the input data. The distance measurement data may comprise at least one value of the at least one distance.

As used herein, the term "at a point in time at which a visual perception of at least one spatial characteristic of the at least one visual stimulus perceived by the person changes" refers to a specific moment at which the change of the distance between the at least one visual stimulus and the eye of the person causes the impression of the person on the spatial resolution, particularly a sharpness, of the at least one visual stimulus perceived by the person to chance. Particularly the distance may change from a first distance at which the person may perceive the at least one stimulus as being fully spatially resolved, also referred to as sharp, to a second distance at which the person may perceive the at least one stimulus as being not fully spatially resolved or sharp, and thereby being blurred; or vice versa. With particular regard to the present invention, it may be pointed out, that a person may perceive the at least one stimulus as being fully spatially resolved or sharp when the at least one stimulus is considered as best resolved or as sharp as possible within the ability of the person. Typically, this ability of the person may be related to the visual acuity of the eye of the person. As generally used, the term "visual acuity" commonly refers to a resolving capacity, specifically a spatial resolving capacity, of a visual system. The term may refer to the ability of the eye to see fine detail. The term may rate the ability of the person to recognize small details with precision. The visual perception of at least one spatial characteristic may change, such as in a typical target detection task, specifically used for determining a visual acuity of an eye of a person, when a perception of a presence and/or an absence of an aspect of the at least one visual stimulus is perceived. Alternatively, or in addition, the visual perception of at least one spatial characteristic may change, such as in a target recognition task, specifically, used for determining a visual acuity of an eye of a person, when a recognition or naming of the at least one visual stimulus by the person may be possible for the first time or impossible for the first time, particularly when the distance may be changed in a monotonous manner. The change may depend on a distance between the eye of the person and the at least one visual stimulus and may particularly thereby be related to visual acuity of the eye of the person. As used herein, the term "at least one spatial characteristic" refers to, particularly in the light of the discussion in the above, to the appearance of at least one detail in the at least one visual stimulus or the entire at least one visual stimulus.

The input data according to step a) is further configured to comprise a position of an entrance pupil of the eye of the person. As generally used, the term "position" refers to a location a place, particularly a position that is considered in regard to the eye of the person. As generally used, the term "entrance pupil" refers to an opening located in a center of an iris of the eye of the person.

According to step b), outcome data is generated. As used herein, the term "outcome data" refers to a set or sequence of data that comprises information determined in at least one step of a computer-implemented method, specifically the computer-implemented method for determining at least one refractive error of the eye of a person. The outcome data may comprise the representative result, particularly determined by the computer-implemented method for determining at least one refractive error of an eye of a person. For generation the outcome data, input data may be considered or evaluated.

The outcome data according to step b) is configured to comprise

- at least one refractive error of the eye of the person, wherein the at least one refractive error of the eye of the person is determined by evaluating the input data, wherein the position of the entrance pupil of the eye of the person is considered when the at least one refractive error of the eye of the person is determined.

As generally used, the term "evaluating" or any grammatical variation thereof refers to a process of interpreting or analyzing data, specifically for determining at least one representative result. The input data may be evaluated for determining the at least one refractive error of the eye of the person.

As generally used, the term "considering" or any grammatical variation thereof refers to a process of taking at least one factor into account, particularly for determining a representative result, more particularly in the evaluation process of the input data. The representative result may depend on the at least one factor in a manner that the result is different for at least two different factors. The position of the entrance pupil of the eye of the person may influence the determined at least one refractive error, particularly as it may be considered as a factor that may influence the distance measurement. The outcome data may be presented to the at least one person, particularly by displaying the outcome data on the at least one screen.

For further details concerning the method, a reference may be made to any aspect and/or any definition according to the present invention as disclosed elsewhere herein.

The input data may comprise measured data, specifically at least one of:

○ the at least one distance between the eye of the person and the at least one visual stimulus displayed to the eye of the person; or
○ the position of the entrance pupil of the eye of the person, and

- wherein the person is a real person.

As used herein the term "measured data" refers to data that is determined from at least one measurement in a physical reality that is particularly extern of a simulation. The physical reality may be different from a simulated reality. As used herein the term "real person" refers to a person that is present in the physical reality that is particularly extern of a simulation. The real person is different from a simulated person.

The method comprises actively determining the measured data. As used herein the term "actively determining" or any grammatical variation thereof refers to the method comprising at least one step in which the measured data is determined. Thereby, the method may operate at least one measurement device, particularly the distance measuring unit.

The refractive error of the eye of the person may be at least one of a value related to:

- a spherical power, particularly dependent on a distance;
- a cylindrical power, particularly dependent on a distance;
- a cylinder axis, particularly dependent on a distance; or
- an addition power, particularly related to a distance for near vision or intermediate vision.

Based on standard ISO 13666:2019 (referred to "Standard" in the following), Section 3.12.2, the term "spherical power", usually abbreviated to "sphere" or "sph", refers to a value of a back vertex power of a spherical-power lens, or for a back vertex power in one of two principal meridians of an astigmatic-power lens, depending on a principal meridian chosen for reference. The spherical power of the at least one eye of the person may be a value related to a "spherical equivalent". As based on the Standard, Section 3.13.7, the term "cylindrical power", usually abbreviated to "cylinder" or "cyl", refers to an algebraic difference between principal powers with power of the principal meridian chosen for reference being subtracted from the other principal power. As based on in the Standard, Section 3.13.8, the term "cylinder axis", usually abbreviated to "cyl axis" or "axis", refers to a direction of the principal meridian of a lens whose vertex power is chosen for reference. As based on in the Standard, Section 3.16.3, the term "addition power", "addition" also abbreviated to "add", refers to a difference between the vertex power of a near portion and the vertex power of a distance portion in a multifocal or power-variation lens. The addition power may further refer to a difference between the vertex power of an intermediate portion and the vertex power of a distance portion in a multifocal or power-variation lens. The addition power may further refer to a single vision lens having a vertex power that is defined for correcting vision for a distance for near vision or intermediate vision.

The person may be using an optical lens for the eye of the person while the input data is generated, particularly wherein the optical lens is having a known prescription. As generally used, the term "optical lens" refers to a visual aid, which may be used for determining and/or correcting a defective vision of the eye of the person. As generally used, the term "prescription" refers to a document that contains at least one piece of information about the optical lens. The optical lens may be the optical lens that the person is generally currently using when the person is being test by applying the method. As used herein, the term "known" refers to the fact that the prescription is available for the method and/or is considered by the method when determining outcome data. Alternatively, or in addition to using an optical lens, the person may have undergone refractive surgery.

The at least one optical lens may be selected from at least one of:

- a spectacle lens;
- a contact lens; or
- an intraocular lens.

Based on the "Standard", 3.5.2, the term "spectacle lens" refers to an optical lens, which is used for determining and/or correcting a defective vision of a wearer of the optical lens, wherein the optical lens is carried in front of the eye of the wearer, thereby avoiding a direct contact with the eye of a wearer. As generally used, the term "contact lens" refers to a lens placed directly on the surface of the eye of the wearer to correct visual defects. As further generally used, the term, "intraocular lens" refers to an artificial lens implanted in the eye of a wearer for correcting the defective vision. The wearer may be a person.

The method may comprise a requesting step, wherein the person is requested to indicate information related to the person in the requesting step. The requesting step may be performed before step a) and/or step b). As used herein, the term "information related to the person" refers to information about at least one body feature of the person, more particularly of the eye of the person, at least one attribute of the person or at least one habit of the person.

The information related to the person may comprises at least one of:

- an age of the person;
- an ethnicity of the person;
- a gender of the person;
- a location of the person at which at least a part of the input data is generated, particularly at which the at least one distance is measured;
- a prescription of the at least one optical lens currently used by the person;
- an indication of the eye of the person for which the at least one refractive error is determined;
- a vertex distance related to a spectacle lens for the eye currently used by the person.

Based on the Standard, 3.2.40, the term "vertex distance" refers to a horizontal distance between the back surface of a spectacle lens and the apex of the cornea, measured with the eyes in the primary position.

The at least one screen may be comprised by at least one of:

- a smartphone;
- a smart watch;
- a desktop computer;
- a laptop;
- a tablet; or
- a smart television.

As generally used, the term "smartphone" refers to a mobile phone having computer functionalities and connectivity and may, additionally, comprise at least one sensor and/or at least one actuator, for example at least one vibration motor. As generally used, the term "smart watch" refers to an electronic wristwatch that has computer functionalities and connectivity and may, additionally, comprise at least one sensor and/or at least one actuator, for example at least one vibration motor. As generally used, the term "smart glasses" refers to wearable spectacles having computer functionalities and connectivity, and may, additionally, add information onto at least one optical lens alongside to what the wearer sees. As further generally used, the term "virtual reality headset" refers to a head-mounted display designed to give a wearer access to a virtual reality. As generally used, the term "desktop computer" refers to a computer in a housing shape suitable for use as a workstation computer on desks. As generally used, the term "laptop" refers to a special type of computer having a screen being movably attached to a housing, wherein the screen can be folded onto the housing. As generally used, the term "tablet" refers to a portable, flat touch-screen computer. As generally used, the term "smart television" refers to a television having computer functionalities and connectivity.

The distance measuring unit may be selected from at least one of:

- an image capturing unit
- a sensor-based capturing unit, particularly selected from at least one of:

    ◦ an infra-red emitter and detector;

    ∘ a LiDAR module; or
    ∘ a Bluetooth module;

- an ultra-wideband unit, particularly comprising at least two communication devices; or
- an independent measurement device, particularly wherein the measurement is performed by the person, specifically wherein the independent measurement device is a ruler.

As generally used, the term "sensor-based capturing unit" refers to a distance sensor and/or proximity sensor that detects signal by using a sensor, particularly for measuring the at least one distance. The detected sensor signal may comprise information related to the at least one distance. The distance measuring unit may, additionally or alternatively, be at least one "image capturing unit" for an image-based measurement of the at least one distance, particularly a camera, specifically of a smartphone, more particularly a back and/or a front camera of the smartphone. For measuring the at least one distance, the at least one distance measuring unit, particularly the at least one image capturing device, more particularly the respective camera, may be calibrated by using standard calibration methods known to the person skilled in the art. The sensor-based capturing unit may be an emitter-detector-unit. As generally used, the term "emitter-detector-unit" refers to a distance sensor and/or a proximity sensor that emit at least one signal by using at least one emitter and detecting this, particularly reflected, at least one signal by using at least one detector. The difference in the emitted signal and the detected signal may comprise information related to the distance, particularly for determining the at least one distance. As generally used, the term "LiDAR scanning module" refers a device suitable for determining the at least one distance by targeting an object or a surface with a laser and measuring the time for the reflected light returning to the receiver. As generally used, the term "independent measurement device" refers to a measurement device that is not comprised by any further unit/and or device used in the method. The independent measurement device may be an analog device, particularly a non-electrical device. The results of the measurements comprising the use of the independent measurement device may be input to the method by using an input device and/or a connection interface.

The at least one visual stimulus may be a type of visual stimulus selected from at least one of:

- a Gabor Patch;
- a grid;
- a circle;
- a point; or
- an optotype.

As generally used, the term "Gabor patch" refers to a grating, usually with a Gaussian envelope, which is known to be particularly useful as visual stimulus for the person's eye. As generally used, the term "optotype" may refer to at least one graphical sign that is known to the person skilled in the art to be used to determine the at least one refractive error of an eye of a person, particularly by considering the visual acuity of the eye of the person. The optotype may be a type of optotype selected from at least one of:

- a Landolt C;
- a Snellen E;
- a Sloan letter;
- an optometric image symbol;
- a letter;
- a text;
- a number;
- a natural picture; or
- a movie scene.

As generally used, the terms "Landolt C", "Snellen E" and "Sloan letter" each refer to a standardized symbol used for testing vision. As generally used, the term "optometric image symbol" refers to a symbol that is typically used for determining the at least one refractive error of an eye of a person, particularly by considering the visual acuity of the eye of the person.

The at least one visual stimulus may be vanishing. As used herein, the term "vanishing" refers to the at least one visual stimulus no longer being perceivable by the eye of the person. A visual stimulus that may be vanishing, particularly an optotype that may be vanishing, more particularly a letter that may be vanishing, may have a pseudo high-pass design so that a mean luminance of the visual stimulus is the same as a mean luminance of the background, thereby the visual stimulus may be considered as vanished as soon as a resolution threshold is reached. This is further described in N. Shah; S. C. Dakin; T. Redmond; R. S. Anderson, Vanishing Optotype Letters as a Clinical Acuity Test: Repeatability and Effect of the Number of Alternatives, ARVO Annual Meeting Abstract, April 2010.

The method may comprise a selecting step, wherein the person is requested to choose at least one characteristic of the at least one visual stimulus. The at least one characteristic to choose may be selected from at least one of:

- a type of the at least one visual stimulus, specifically a type of the optotype;
- a color of the at least one visual stimulus;
- an orientation of the at least one visual stimulus;
- a spatial frequency of the at least one of the visual stimulus;
- a size of the at least one of the visual stimulus;
- a contrast level of the at least one of the visual stimulus;
- a polarization of the at least one of the visual stimulus;
- a flicker frequency of the at least one of the visual stimulus;
- a display time of the at least one of the visual stimulus; or
- a shape of the at least one of the visual stimulus.

As generally used, the term "spatial frequency" refers to a reciprocal value of a spatial distance reflecting a spatial period of repetition in the at least one visual stimulus. As generally used, the term "contrast level" refers to a value reflecting a luminance level of at least one visual stimulus. As generally used, the term "polarization" refers to a property of transverse waves, particularly light waves, that specifies a geometrical orientation of at least one oscillation. As generally used, the term "flicker frequency" refers to a degree reflecting a reciprocal value of a temporal period of repetition of the at least one visual stimulus. As used herein, the term "display time" refers to a period of time during which the at least one visual stimulus is displayed on the at least one screen.

A further eye of the person for which the at least one refractive error is not determined may be shielded from the at least one visual stimulus displayed to the eye of the person. As used herein, the term "shielding" or any grammatical variation thereof refers to the further eye being prevented from perceiving the at least one visual stimulus, particularly by blocking or redirecting at least a part or the full field of view of the further eye so that light originating from the at least one visual stimulus cannot imping on the at least one eye. The term "field of view" as used herein refers to an extent of the observable world that is observable, particularly by the further eye of the person. The field of view may be considered as a solid angle through which an eye, particularly the further eye, is sensitive to the at least one visual stimulus. The further eye of the person may be shielded by at least one of:

- closing the further eye; or
- covering the further eye, specifically by using a cover, preferably a cardboard or a hand of the person or of a further person.

The distance between the eye of the person and the at least one visual stimulus displayed to the eye of the person may be changed by at least one of:

- moving the eye of the person towards the at least one screen;
- moving the at least one screen towards the eye of the person; or
- changing the geometry of the field of view of the eye of the person between the eye and the at least one visual stimulus, particularly by using at least one of:

    o at least one optical lens; or
    o at least one mirror;

- changing a virtual reality.

As generally used, the term "optical lens" refers to a transparent unit, particularly an item, wherein at least one surface of the object is curved, particularly in a spherical manner. Incident light may be refracted at the at least one surface of the optical lens, particularly depending on the wavelength of the incident light. As generally used, the term "mirror" refers to an optical unit having a reflective surface for reflecting at least a portion of light impinging on the reflective surface, particularly at least a portion of the incident light. A typical mirror may reflect at least 5% of the light that is incident on the mirror. Further typically a mirror may, preferably, reflect at least 50% or, more preferably, at least 90% of the light that is incident on the mirror. Relevant light for the invention may be visible light, particularly having a wavelength between 380 nm and 760 nm (based on ISO 21348, *Definitions of Solar Irradiance Spectral Categories*). As generally used, the term "virtual reality" refers to a simulated experience, particularly a simulated environment.

The distance between the eye of the person and the at least one visual stimulus displayed to the eye of the person may be modified by, particularly monotonously, decreasing the distance. As generally used, the term "monotonous" refers to a uniform and/or consistent motion. The motion may be considered as monotonous when a direction of the motion is constant and/or remains, particularly unchanged. Thereby the distance between the eye of the person and the at least one visual stimulus may be changed by being constantly increased or being constantly decreased. The direction in which the distance changes may thus be considered as remaining constant. The speed of the change of the distance may fluctuate.

The distance between the eye of the person and the at least one visual stimulus displayed to the eye of the person may be decreased from a first starting position. The person may be holding the at least one screen in at least one hand and may be actively changing the distance, particularly by increasing, or decreasing the distance, between the eye of the person and the at least one visual stimulus displayed to the eye of the person. The person may be holding the at least one screen in at least one hand, wherein at least one arm comprising the at least one hand may be, particularly fully, extended at the first starting position. Alternatively or in addition, the at least one screen may be fixedly positioned, particularly on a stable ground, in front of the person and the person is actively changing the distance between the eye of the person and the at least one visual stimulus displayed to the eye of the person.

The distance between the eye of the person and the at least one visual stimulus displayed to the eye of the person may be modified by, particularly monotonously, increasing the distance. The distance between the eye of the person and the at least one visual stimulus displayed to the eye of the person may be increased from a second starting position. The person may be holding the at least one screen in at least one hand, wherein the at least one screen may be positioned in close proximity of the eye of the person in the second starting position.

A plurality of visual stimuli may be displayed to the eye of the person, particularly wherein the plurality of visual stimuli may be displayed on the at least one screen. At least two visual stimuli or each visual stimulus of the plurality of visual stimuli may differ in at least one of:

- a type, specifically a type of the optotype;
- a color;
- an orientation;
- a spatial frequency;
- a size;
- a contrast level;
- a polarization;
- a flicker frequency;
- a display time; or
- a shape.

At least two visual stimuli or each visual stimulus of the plurality of visual stimuli may have a different display time. Thereby, a start of the display time and/or an end of the display time of the at least two visual stimuli or each visual stimulus may be different. The at least two visual stimuli or each visual stimulus may or may not be, particularly at least partly, displayed at the same time. The at least one distance may be measured at a point in time at which the visual perception of the at least one spatial characteristic of a fraction of the plurality of the visual stimuli perceived by the person changes, particularly due to the change of the distance between the distance between the eye of the person and the at least one visual stimulus displayed to the eye of the person. The term "fraction" may refer to a total number of visual stimuli that is perceived as sharp or blurred divided by the total number of visual stimuli that is displayed. The fraction may correspond to a maximum of 20%, of 40%, of 60% or of 80% of the plurality of visual stimuli. The fraction corresponding to 60% may particularly be preferred, specifically as this fraction may be in line with ISO 8596:2017, *Ophthalmic optics - Visual acuity testing - Standard and clinical optotypes and their presentation.*

At least one of:

- a number of the plurality of the visual stimuli displayed to the eye of the person;
- a type of at least one visual stimulus of the plurality of visual stimuli displayed to the eye of the person, specifically a type of the optotype;
- a color of at least one visual stimulus of the plurality of visual stimuli displayed to the eye of the person;
- an orientation of at least one visual stimulus of the plurality of visual stimuli displayed to the eye of the person;
- a spatial frequency of at least one visual stimulus of the plurality of visual stimuli displayed to the eye of the person;
- a size of at least one visual stimulus of the plurality of visual stimuli displayed to the eye of the person;
- a contrast level of at least one visual stimulus of the plurality of visual stimuli displayed to the eye of the person;
- a polarization of at least one visual stimulus of the plurality of visual stimuli displayed to the eye of the person;
- a flicker frequency of at least one visual stimulus of the plurality of visual stimuli displayed to the eye of the person; or
- a display time of at least one visual stimulus of the plurality of visual stimuli displayed to the eye of the person;

may depend on a minimum angle of resolution, MAR, of the eye of the person. As generally used, the term "minimum angle of resolution" refers to a reciprocal of the acuity number, which particularly may be a rate for the ability of the person to recognize small details with precision.

The visual perception of the at least one spatial characteristic of the at least one visual stimulus perceived by the person may change from blurred to sharp or from sharp to blurred at the point in time. As generally used, the term "sharp" refers to a state in which an object is spatially resolved optimally in regard to a spatially resolving capacity of a visual system. In that sense the person may consider the at least one visual stimulus as sharp, when the person considers the ability of the eye to see fine detail in the at least one visual stimulus is met as good as possible. The term "sharp" may thus refer to a state in which the distinguishability of details is considered optimal, particularly to the ability of the person. An object that is considered sharp may not be considered blurred. In the context of the invention, the at least one visual stimulus may be considered sharp by the person, when the person can identify the highest number of details of the at least one visual stimulus to the person's ability. The absolute amount of details that may be identified may vary between different persons, particularly depending on the visual acuity of the eye of the person. As generally used, the term "blurred" may refer to a state in which an object is spatially not resolved optimally in regard to a spatially resolving capacity of a visual system. In that sense the person may consider the at least one visual stimulus as blurred, when the person considers the ability of the eye to see fine detail in the at least one visual stimulus is not met as good as possible. The term "blurred" may thus refer to a state of imprecision, indeterminacy, or uncertainty of a visually perceived object. In the context of the invention, the at least one visual stimulus may be considered blurred by the person, when the person cannot identify the highest number of details of the at least one visual stimulus, particularly considering the ability of the person.

It may be determined that the visual perception of the at least one spatial characteristic of the at least one visual stimulus perceived by the person changes and that the at least one distance is to be recorded by at least one of:

- an indication of the person, particularly wherein the indication is given by at least one of:

    o a visual signal, particularly a gesture;
    o an audio signal, particularly a voice input, specifically given by using a microphone;
    o a tactile signal, particularly by using at least one of:

        ▪ a keypad; or
        ▪ a touch pad;

- an indication of a further person, particularly wherein the indication is given by at least one of:

    o a visual signal, particularly a gesture;
    ○ an audio signal, particularly a voice input, specifically given by using a microphone;
    ○ a tactile signal, particularly by using at least one of:

        ▪ a keypad; or
        ▪ a touch pad;

- a continuous distance measurement and an automatic threshold estimation or calculation; or
- staircase method and/or forced choice method.

As used herein, the term "indication" refers to a detail or an information directly and/or indirectly related to the feature the indication is about. The indication may thus be the feature itself or a paraphrase of the feature. As used herein, the term "visual signal" refers to a sign perceptible by a sense of seeing. The term "audio signal" is refers to a sound perceptible by a sense of hearing. The term "tactile signal" refers to an impression that is perceived by a haptic sensation, for example haptic sensations such as, but not limited to, tickling, a touch, a movement, a vibration, a temperature, a pressure and/or a tension. As generally used, the term "continuous distance measurement and an automatic threshold estimation or calculation" refers to a test procedure that uses a continuous distance measurement for positioning the patient, particularly as close as possible to a visual stimulus in a manner that the visual stimulus may be seen or as far as possible to a visual stimulus in a manner that the visual stimulus may be seen, particularly as spatially optimally resolved. An individual visual acuity value may then be determined by the measured distance, a stimulus size, and a threshold determination. As generally used, the term "staircase method" refers to method involving upward and downward movements of a size or spatial characteristic of the visual stimulus and/or the distance. Thereby, a test-retest variability may be improved. As regards the term "forced choice method" reference is made to the definition of the term "forced choice principle". In a forced choice method, the forced choice principle may be used.

The at least one recorded distance may correspond to at least one of:

- a near point distance; or
- a far point distance;

of the eye of the person. Based on DIN 5340, *Begriffe der physiologischen Optik,* April 1998 (referred to as the "DIN 5340" in the following), the term "near point distance" refers to a distance of a near point from the object-side main point of the eye, according to No. 288 of DIN 5340. Based on DIN 5340, No. 287, the term "near point" is an adjustment point at the highest refractive index of the optical system of the eye. Based on DIN 5340, No. 152, the term "far point distance" refers to a distance of a far point from the object-side main point of the eye. Based on DIN 5340, No. 151, the term "far point" refers to an adjustment point at the lowest refractive index of the optical system of the eye.

It may be determined that the at least one recorded distance corresponds to a near point distance by at least one of:

- an indication of the person;
- an indication of the further person;
- by considering if the distance between the eye of the person and the at least one visual stimulus is changed by increasing or decreasing;
- by considering an age of the person; or
- by considering a table.

It may be determined that the at least one recorded distance corresponds to a far point distance by at least one of:

- an indication of the person;
- an indication of the further person;
- by considering the direction in which the distance between the eye of the person and the at least one visual stimulus is changed by increasing or decreasing;
- by considering an age of the person; or
- by considering a table.

Typically, in case the distance between the eye and the at least one visual stimulus is increased, the distance at which the visual perception of the at least one spatial characteristic of the at least one visual stimulus changes from blurred to sharp may be considered as the near point distance. In case the distance is between the eye and the at least one visual stimulus is further increased, the distance at which the visual perception of the at least one spatial characteristic of the at least one visual stimulus changes from sharp to blurred may be considered as the far point distance. In case the distance is between the eye and the at least one visual stimulus is decreased, the distance at which the visual perception of the at least one spatial characteristic of the at least one visual stimulus changes from blurred to sharp may be considered as the far point distance. In case the distance is between the eye and the at least one visual stimulus is further decreased, the distance at which the visual perception of the at least one spatial characteristic of the at least one visual stimulus changes from sharp to blurred may be considered as the near point distance.

A table that may be used is given in the textbook Augenheilkunde by A.J. Augustin, 3rd edition, Springer, 2007 on p. 591. In this table, typical near point distances depending on the age of the person are listed.

The at least one distance between the eye of the person and the at least one visual stimulus may be continuously measured. As generally used, the term "continuous" or any grammatical variation thereof refers to a circumstance that is ongoing, uninterrupted and/or uniformly continuing. For measuring the distance continuously, a plurality of repeated distance measurements may be performed with a predetermined time interval between each repetition. Alternatively, for measuring the distance continuously, the distance may be measured all the time or as often as technically possible. The size of the at least one visual stimulus displayed to the eye of the person may be adjusted depending on the at least one distance continuously measured between the eye of the person and the at least one visual stimulus. The size of the at least one visual stimulus displayed to the eye of the person may be adjusted in a manner that the person perceives the visual stimulus in the same size independently from the at least one distance continuously measured between the eye of the person and the at least one visual stimulus.

Step a) may define a measurement cycle. A plurality of measurement cycles may be performed. Step b) may define an evaluation cycle. A plurality of measurement cycles may be performed, wherein in each measurement cycle the at least one distance may be recorded, wherein any one of the at least one recorded distance may be considered in the evaluation cycle, particularly in a final evaluation cycle.

The method may further comprise an astigmatism determining step; wherein it may be determined if the eye of the person suffers from astigmatism by displaying a visual stimulus configured for determining an astigmatism, particularly being a type of visual stimulus selected from at least one of:

- at least one line or a plurality of lines, particularly wherein the plurality of lines is grouped, specifically grouped in a manner according to which at least two lines of the plurality of lines are oriented in different directions;
- a sun wheel or an astigmatism dial;
- a Raubitschek figure;
- a Gabor patch;
- a grid;
- a cross;
- a Snellen E; or
- a Landolt C;

and requesting an indication of the person for testing if the eye of the person suffers from astigmatism.

The method may further comprise an astigmatism determining step, wherein the astigmatism may be determined by using a forced choice principle. As generally used, the term "forced choice principle" refers to a trial procedure in which the person being tested is necessarily required to give an indication related to or about a subject to be tested. The person may necessarily give an indication even in case that the person is unsure. Thereby, the person may have to give a guessed indication rather than an indication that the person is unsure. Typical indications may be answers such as "yes" or "no".

The method may further comprise a cylinder axis determining step, wherein the cylinder axis may be determined by at least one of:

- displaying a visual stimulus configured for determining a cylinder axis, particularly selected from at least one of:

  ○ a sun wheel; or
  ○ an astigmatism dial;
  ○ at least one line or a plurality of lines, particularly wherein the plurality of lines is grouped, specifically wherein the line is oriented in different directions;
  ○ a Raubitschek figure;
  ○ a Gabor patch;
  ○ a grid;
  ○ a cross;
  ○ a Snellen E; or
  ○ a Landolt C;

  and requesting an indication of the person for determining the cylinder axis;
- considering a known cylinder axis value from a known prescription.

The visual stimulus may be oriented in a manner defined by the determined cylinder axis, particularly considering the meridians defined by the cylinder axis.

At least one of:

- a distal far point distance;
- a proximal far point distance;
- a distal near point distance; or
- a proximal near point distance;

may be recorded.

The terms "distal" and "proximal" refer to the principal meridians of an astigmatic eye. As generally used, the term "distal" refers to the steepest meridian. As generally used, the term "distal" refers to the flattest meridian. The degree between the steepest meridian and the flattest meridian may be 90°. There may be a different degree between the steepest meridian and the flattest meridian, such as 91°.

The eye of the person may suffer from astigmatism, wherein at least two measurement cycles may be performed, wherein in a first measurement cycle the visual stimulus may be oriented in a manner configured for a recording of at least one of:

- a distal far point distance; or
- a distal near point distance;

and wherein in a second measurement cycle the visual stimulus may be oriented in a manner configured for a recording of at least one of:

- a proximal far point distance; or
- a proximal near point distance.

Thereby, in the first measurement cycle a distal far point distance or the distal near point distance may be determined and in the second measurement cycle the proximal far point distance or the proximal near point distance may be determined. In a third measurement cycle the remaining distal distance, particularly the distal near point distance or the distal far point distance may be determined. In a fourth measurement cycle the remaining proximal distance, particularly the proximal near point distance or the proximal far point distance may be determined. There may be further sequences to determine the distances. These further sequences may involve more than four measurement cycles.

The eye of the person may suffer from astigmatism, wherein at least three measurement cycles are performed, wherein in each measurement cycle a different meridian may be used, wherein a first meridian may be at 0°, wherein a second meridian may be at 60° and wherein a third meridian may be at 120°. The first meridian may be defined arbitrarily in a manner that any offset may be compensated. It may also be possible to select the meridians in a manner having a different offset but the same ratio, particularly a 60° step size between different meridians. A larger number of meridians may further be selected, particularly four, five or six meridians. Alternatively, an even larger number of meridians may be selected.

Considering the position of the entrance pupil of the eye of the person comprises considering the position of the entrance pupil in relation to at least one further portion of the eye of the person. The considered at least one further portion of the eye of the person is a cornea of the eye of the person. As generally used, the term "cornea" refers to an anterior part of the outer skin of the eye. Typically, the cornea is vitreous and performs much of the refraction of light incident in the eye of the person. The cornea may the frontal closure of the eye.

According to the invention, considering the position of the entrance pupil in relation to at least one further portion of the eye of the person comprises considering a distance between the entrance pupil, particularly a center of the entrance pupil, and the cornea. The distance between the entrance pupil and the cornea is added to the distance between the eye of the person and the at least one visual stimulus for considering the position of the entrance pupil.

The distance may be parallel and/or coaxial with respect to the line of sight of the eye of the person. Based on the Standard, Section 3.2.24, the term "line of sight" refers to a path from a point of interest, i.e., a point of fixation, in object space to a center of the entrance pupil of the eye of the person, The line of sight may, further, comprise a continuation in image space from a center of an exit pupil to a retinal point of fixation, generally the foveola, in the eye of the person. The distance between the entrance pupil and the cornea may be determined by considering an average radius of the cornea of the eye of the person, particularly by dividing the average radius of the cornea of the eye of the person by factor of 2. As generally used the term "average radius" refers to the average distance from a center, particularly the center of the eye of the person, to a surface, particularly the cornea of the eye. The average radius may be based on a plurality of distances, especially measured distances.

At least one characteristic of the cornea may be measured for considering the position of the entrance pupil. The at least one measured characteristic may be a curvature of the cornea, particularly average radius of the cornea. The method may comprise a characteristic of the cornea measuring step, wherein the characteristic of the cornea may be measured by using at least one of:

- an ophthalmometer or keratometer, particularly for determining a corneal topography; more particularly considering at least one of:

  ◦ at least one reflection on the cornea; or
  ◦ a keratometry method, particularly involving the use of a video, more specifically a Placido method;
  ◦ a smartphone-based video keratometry

- tomography; particularly considering at least one of:

  ◦ optical coherence tomography, OCT; or
  ◦ a Scheimpflug principle.

The at least one characteristic of the cornea may be measured at least one of:

- before a first measurement;
- during a measurement cycle;
- after a measurement cycle; or
- after a final measurement cycle.

The at least one characteristic may be measured multiple times.

For determining the refractive error of the eye of the person an age dependent accommodation amplitude AA or an age dependent near point distance, aNPD, may be considered, particularly for correcting the measured near point distance, NPD. As generally used, the term "accommodation amplitude" refers to a maximum potential of an eye to increase in optical power for adjusting its focus. It may refer to a certain range of object distances for which the retinal image may be as sharply focused as possible. The accommodation amplitude may be different for different ages of the person. Thereby, the accommodation amplitude may be "age dependent". As the accommodation amplitude may be age dependent, the near point distance may be age dependent.

The age dependent accommodation amplitude AA in meter for a person being 52 years old or being younger than 52 years old may be determined by using equation (1)

$$AA = 15.6 - \frac{1}{3} * \text{age}, \qquad (1)$$

wherein age is the age of the person. The age dependent accommodation amplitude AA may have a value of 0 for a person being older than 52. The age dependent accommodation amplitude AA is determined from reading a table. The age dependent near point distance aNPD may be determined from reading a table. Again, a table that may be used is given in the textbook Augenheilkunde by A.J. Augustin, 3rd edition, Springer, 2007 on p. 591. In this table, typical near point distances depending on the age of the person are listed.

For determining the refractive error of the eye of the person a parameter K may be considered, wherein the parameter K depends on a first color of the visual stimulus and a second color of the background on which the visual stimulus is displayed. The "parameter K" may be considered as a correction factor for a chromatic aberration, particularly a longitudinal chromatic aberration. As generally used, the term "chromatic aberration" refers an aberration or image defect of an optical lens, particularly the eye, specifically occurring when light of different wavelengths or colors is refracted to different extents. The term "longitudinal chromatic aberration" refers to the fact that a focus of a lens depends on the wavelength of the incident light in a manner that different colors create different focal planes.

The parameter K may depend on at least one spectrum emitted by the at least one screen for displaying at least one of:

- the background; or
- the visual stimulus.

As generally used, the term "background" refers to a part of the display that appears distant to an observer. The at least one screen may display the background and the at least one visual stimulus at the same time. The at least one visual stimulus may be placed on the background. The at least one visual stimulus may be surrounded by the background.

It may be determined that the eye of the person suffers from myopia by at least one of:

- a far vision test, particularly a refraction test specifically an objective or subjective refraction test; or
- a duochrome test.

As generally used, the term "myopia" refers to an optical defect of an eye, wherein the eye focuses light in front of the retina. As generally used, the term "far vision test" refers to an examination of the eye conducted for testing the far vision of the person. Such a test may particularly be a "refraction test". In a refraction test, typically, the person is asked to look at an eye chart, while different optical lenses are moved in the field of view of the tested eye. A refraction test may, typically, be performed by an ophthalmologist or an optometrist. Typically, a "subjective" refraction test may be based on trial and error testing and an eye chart. In such a test, the person may have to give an indication about a test item. On the other hand, an "objective" refraction test refers to an examination of the eye of the person, in which the refraction results may be determined without relying on an indication or a response of the patient. As generally used, the term "duochrome test" refers to an examination of the eye that is typically used for redefining the final sphere in refraction. Thereby, the duochrome test may makes use of a longitudinal chromatic aberration of the eye.

The spherical power SPH in diopter may be determined by using equation (2)

$$SPH = -\frac{1}{FPD+dEP}, \qquad (2)$$

FPD is the far point distance and dEP is the distance between the entrance pupil and the cornea. The spherical power SPH in diopter may be determined by using equation (3)

$$SPH = -\frac{1}{FPD+dEP} + K, \qquad (3)$$

wherein FPD is the far point distance, dEP is the distance between the entrance pupil and the cornea as well as K is a parameter dependent on the first color of the visual stimulus and the second color of the background on which the visual stimulus is displayed.

The eye of the person may further suffer from astigmatism, wherein for calculating the spherical power the far point distance FPD may be the distal far point distance, dFPD. The cylindrical power CYL may be determined by using equation (4)

$$CYL = -\left(\frac{1}{pFPD} - \frac{1}{dFPD}\right), \qquad (4)$$

wherein pFPD is the proximal far point distance and dFPD is the distal far point distance.

The cylindrical power CYL may be determined by using equation (5)

$$CYL = -\left(\frac{1}{pFPD+dEP} - \frac{1}{dFPD+dEP}\right), \qquad (5)$$

wherein pFPD is the proximal far point distance, dFPD is the distal far point distance and dEP is the distance between the entrance pupil and the cornea.

The cylindrical power CYL may be determined by using equation (6)

$$CYL = -\left(\frac{1}{pFPD+dEP} - \frac{1}{dFPD+dEP}\right) + K, \qquad (6)$$

wherein pFPD is the proximal far point distance, dFPD is the distal far point distance, dEP is the distance between the entrance pupil and the cornea and K is the correction factor of the longitudinal chromatic aberration.

It may be determined that the eye of the person suffers from hyperopia by at least one of:

- a near vision test, particularly a refraction test specifically an objective or subjective refraction test;
- a duochrome test;
- a reading task;
- a consideration of an age of the person; or
- considering a known prescription.

As generally used, the term "hyperopia" refers to an optical defect of an eye, wherein the eye focuses light in behind the retina. In case the eye has still sufficient accommodation, the eye lens may be able to compensate such a hyperopic optical defect to a certain degree. As generally used, the term "near vision test" refers to an examination of the eye conducted for testing the near vision of the person.

The spherical power SPH in diopter may be determined by using equation (7)

$$SPH = -\frac{1}{NPD+dEP} + AA, \qquad (7)$$

wherein NPD is the near point distance, dEP is the distance between the entrance pupil and the cornea as well as AA is the age dependent accommodation amplitude.

The spherical power SPH in diopter may be determined by using equation (8)

$$SPH = -\frac{1}{NPD+dEP} + AA + K, \qquad (8)$$

wherein NPD is the near point distance, dEP is the distance between the entrance pupil and the cornea., AA is an age dependent accommodation amplitude and K is a parameter dependent on the first color of the visual stimulus and the second color of the background on which the visual stimulus is displayed.

Instead of the age dependent accommodation amplitude AA an age dependent near point distance aNPD is considered. The age dependent near point distance aNPD may be subtracted from the near point distance NPD.

The eye of the person may further suffer from astigmatism, wherein for calculating the spherical power the near point distance NPD may be the distal near point distance dNPD.

The cylindrical power CYL may be determined by using equation (9)

$$CYL = -\left(\frac{1}{pNPD} - \frac{1}{dNPD}\right), \qquad (9)$$

wherein pNPD is the proximal near point distance and dNPD is the distal near point distance.

The cylindrical power CYL may be determined by using equation (10)

$$CYL = -\left(\frac{1}{pFPD+dEP} - \frac{1}{dFPD+dEP}\right), \qquad (10)$$

wherein pFPD is the proximal far point distance, dFPD is the distal far point distance and dEP is the distance between the entrance pupil and the cornea.

The cylindrical power CYL may be determined by using equation (11)

$$CYL = -\left(\frac{1}{pFPD+dEP} - \frac{1}{dFPD+dEP}\right) + K, \qquad (11)$$

wherein pFPD is the proximal far point distance, dFPD is the distal far point distance, dEP is the distance between the entrance pupil and the cornea and K is the correction factor of the longitudinal chromatic aberration.

For the eye of the person suffering from myopia and astigmatism a cylinder axis AXIS may be determined for

$$0° < \text{degree of pFPD} < 90°,$$

wherein pFPD is the proximal far point distance, by using equation (12)

$$AXIS = 90° - \text{degree of pFPD or}$$

$$AXIS = 270° - \text{degree of dFPD}, \qquad (12)$$

wherein dFPD is the distal far point distance.

For the eye of the person suffering from hyperopia and astigmatism a cylinder axis AXIS may be determined for

$$0° < \text{degree of pNPD} < 90°,$$

wherein pNPD is the proximal near point distance, by using equation (13)

$$AXIS = 90° - \text{degree of pNPD or}$$

$$AXIS = 270° - \text{degree of dNPD}, \qquad (13)$$

wherein dNPD is the distal near point distance.

According to a further aspect of the present invention, a computer program according to claim 7 is disclosed, comprising instructions which, when the program is executed by a computer, cause the computer to carry out the method as discussed in the above. As generally used, the term "computer program" refers to at least one executable instruction for at least one programmable apparatus, specifically a computer, preferably a sequence of executable instructions, for processing and/or solving of at least one function and/or at least one task and/or at least one problem by using at least one programmable apparatus or device, specifically a computer, preferably for performing some or all steps of any one of the methods as described within the present invention. Typically, instructions are combined to a computer program code and/or provided in a programming language. A computer program is typically processed by using a processing device comprised by the at least one computer. For this purpose, the computer program may be running on the computer. The computer program code may be provided on a data storage medium or a separate device such as an optical storage medium, e.g., on a compact disc, directly on a computer or data processing device, or via a network, such as via an in-house network or via internet. For further details concerning the computer program, a reference may be made to any aspect and/or any definition according to the present invention as disclosed elsewhere herein. A further aspect not forming part of the present invention relates to a field device for generating input data for determining at least one refractive error of an eye of a person, wherein the field device is configured to carry out a computer-implemented method for determining at least one refractive error of an eye of a person, comprising the following steps:

a) generating input data configured to comprise

- at least one distance between the eye of the person and at least one visual stimulus displayed to the eye of the person, wherein the at least one distance between the eye of the person is determined by

  ○ displaying to the eye of the person at least one visual stimulus on at least one screen while the distance between the eye of the person and the at least one visual stimulus displayed to the eye of the person is changed;
  ○ recording the at least one at least one distance by using a distance measuring unit at a point in time at which a visual perception of at least one spatial characteristic of the at least one visual stimulus perceived by the person changes;

- a position of an entrance pupil of the eye of the person;

wherein the input data set is forwarded to a remote device by using a connection interface for determining at least one refractive error of the eye of the person by considering the position of an entrance pupil of the eye of the person. For further details concerning the field device, a reference may be made to any aspect and/or any definition according to the present invention as disclosed elsewhere herein.

As used herein, the term "field device" refers to a device that is used for generating the input data. The person may have access to the field device and/or the person may use the field device, particularly for generating the input data. Alternatively, a further person may use the field device on the person, particularly for generating the input data. The field device may be operable without a remote device. As used herein, the term "remote device" refers to a device that is used for generating the outcome data. The person may not have access to the field device and/or the person may not or may only indirectly use the field device, particularly for generating the outcome data. The remote device may be owned, used and/or controlled by a third party, for example a company or a further person. The remote device may be operable without the field device. The field device may transmit the input data to a specific remote device depending on at least one circumstance, such as a date, a day, a low load of the specific remote device, particularly in comparison to at least one alternative remote device, low costs for running the specific remote device, particularly in comparison to at least one alternative remote device, and so on. The specific remote device may not be directly selected by the field device but rather a further device may specify to which specific remote device the input data may be transmitted from the field device. The generation of the outcome data may involve a use of several different entities of the remote device. At least one entity may generate intermediate data and transmit the intermediate data by using a connection interface to at least one further entity. The outcome data may be transmitted from the remote device to the field device, particularly by using a data carrier signal carrying the outcome data. Thereby, the person may be able to analyze the outcome data, specifically the at least one refractive error of the eye of the person.

As generally used, the term "connection interface" refers an item or an element forming a boundary configured for transmitting information or data, particularly input data, or outcome data. In particular, the connection interface may be configured for transmitting information from a computational device, e.g. a computer, such as to forward input data or output data, e.g. onto another device. Additionally, or alternatively, the connection interface may be configured for transmitting information onto a computational device, e.g. onto a computer, such as to receive information. The connection interface may specifically be configured for transmitting or exchanging information. In particular, the connection interface may provide a data transfer connection, e.g. Bluetooth, NFC, or inductive coupling. As an example, the connection interface may be or may comprise at least one port comprising one or more of a network or internet port, a USB-port, and a disk drive.

The field device may be at least one of:

- a smartphone;
- a smart watch;
- a desktop computer;
- a laptop;

- a tablet; or
- a smart television.

According to a further aspect, the present invention relates to a remote device for generating outcome data for determining at least one refractive error of an eye of a person according to claim 8, wherein the device is configured to carry out a computer-implemented method for determining at least one refractive error of an eye of a person, wherein the remote device is receiving input data provided by a field device by using a connection interface, wherein the method is comprising the steps defined in claim 8 including, inter alia, the following step:
b) generating outcome data configured to comprise

- at least one refractive error of the eye of the person, wherein the at least one refractive error of the eye of the person is determined by evaluating the input data, wherein a position of an entrance pupil of the eye of the person is considered when the at least one refractive error of the eye of the person is determined.

For further details concerning the remote device, a reference may be made to any aspect and/or any definition according to the present invention as disclosed elsewhere herein.
As generally used, the term "processing device" refers to an arbitrary logic circuitry configured for performing basic operations of a computer or system, and/or, generally, to a device which is configured for performing calculations or logic operations. Herein, the processing device may be a single device. As an alternative, the processing device may comprise a plurality of elements which are located on more than a single location, wherein at least two elements located on at least two different locations are configured to communicate with each other by using at least one connection interface, especially at least one connection interface as described elsewhere herein in more detail. In particular, the processing unit may be configured for processing basic instructions that drive the computer or system. As an example, the processing unit may comprise at least one arithmetic logic unit (ALU), at least one floating-point unit (FPU), such as a math co-processor or a numeric coprocessor, a plurality of registers, specifically registers configured for supplying operands to the ALU and storing results of operations, and a memory, such as an L1 and L2 cache memory. In particular, the processing unit may be a multi-core processor. Specifically, the processing unit may be or may comprise a central processing unit (CPU). Additionally, or alternatively, the processing unit may be or may comprise a microprocessor, thus specifically the processing unit's elements may be contained in one single integrated circuitry (IC) chip. Additionally, or alternatively, the processing unit may be or may comprise one or more application-specific integrated circuits (ASICs) and/or one or more field-programmable gate arrays (FPGAs). The processing unit specifically may be configured, such as by software programming, for performing one or more evaluation operations.
The remote device may be at least one of:

- a server; or
- a cloud server.

As generally used, the term "server" refers to device for performing at least one task for a further device connected to the server for transmitting data, particularly connected by a network such as the internet or a private network. As generally used, the term "cloud server" refers to a server that is accessible via a public network such as the internet. A cloud server may particularly be owned by a third party and offer third party service such as evaluating the input data for generating the outcome data and thereby determining the at least one refractive error of the eye of the person.
The connecting interface may be selected from at least one of:

- a network interface controller; or
- a transmitter.

As generally used, the term "network interface controller" refers to a computer hardware component that connects a computer to a computer network. As generally used, the term "transmitter" refers to an electronic device, which produces electromagnetic waves with an antenna.
A further aspect not forming part of the present invention relates to a data carrier signal carrying outcome data which are generated by a method which comprises generating the outcome data. For further details concerning the data carrier signal, a reference may be made to any aspect and/or any definition according to the present invention as disclosed elsewhere herein. As generally used, the term "data carrier signal" refers to an electronic signal comprising information. The data carrier signal may be provided on a computer-readable storage medium. As used herein, the term "computer-readable storage medium" specifically may refer to a non-transitory data storage means, such as a hardware storage medium having stored thereon computer-executable instructions.
According to a further aspect, the present invention relates to a determining device for determining at least one refractive error of an eye of a person according to claim 9, wherein the device is configured to carry out a computer-implemented method for determining at least one refractive error of an eye of a person, wherein the method is comprising the steps defined in claim 9 including, inter alia, the following steps

a) generating input data configured to comprise

- at least one distance between the eye of the person and at least one visual stimulus displayed to the eye of the person, wherein the at least one distance between the eye of the person is determined by

  ∘ displaying to the eye of the person at least one visual stimulus on at least one screen while the distance between the eye of the person and the at least one visual stimulus displayed to the eye of the person is changed;
  ∘ recording the at least one at least one distance by using a distance measuring unit at a point in time at which a visual perception of at least one spatial characteristic of the at least one visual stimulus perceived by the person changes;

- a position of an entrance pupil of the eye of the person;

b) generating outcome data configured to comprise

- at least one refractive error of the eye of the person, wherein the at least one refractive error of the eye of the person is determined by evaluating the input data, wherein the position of the entrance pupil of the eye of the person is considered when the at least one refractive error of the eye of the person is determined.

For further details concerning the remote device, a reference may be made to any aspect and/or any definition according to the present invention as disclosed elsewhere herein.
The determining device may be at least one of:

- a smartphone;
- a smart watch;
- a desktop computer;
- a laptop;
- a tablet; or
- a smart television.

The distance measuring unit may be at least one of:

- an image capturing unit
- a sensor-based capturing unit, particularly selected from at least one of:

  ∘ an infra-red emitter and detector;
  ∘ a LiDAR module; or
  ∘ a Bluetooth module;

- an ultra-wideband unit, particularly comprising at least two communication devices; or
- an independent measurement device, particularly wherein the measurement is performed by the person, specifically wherein the independent measurement device is a ruler.

According to a further aspect, the present invention relates to a method for producing a geometrical model of at least one spectacle lens for manufacturing of the at least one spectacle lens according to claim 10, wherein producing the geometrical model comprise, inter alia,

- producing a geometrical model of at least one spectacle lens for at least one eye of a person by using data related to at least one refractive value; and
- determining the data related to the at least one refractive value by carrying out a computer-implemented method for determining at least one refractive error of an eye of a person in a vision testing procedure, wherein the vision testing procedure comprises at least one test cycle, preferably at least two subsequent test cycles, wherein the test cycle comprises at least the following steps:

a) generating input data configured to comprise

- at least one distance between the eye of the person and at least one visual stimulus displayed to the eye of the person, wherein the at least one distance between the eye of the person is determined by

  ∘ displaying to the eye of the person at least one visual stimulus on at least one screen while the distance between the eye of the person and the at least one visual stimulus displayed to the eye of the person is changed;
  ∘ recording the at least one at least one distance by using a distance measuring unit at a point in time at which a visual perception of at least one spatial characteristic of the at least one visual stimulus perceived by the person changes;

- a position of an entrance pupil of the eye of the person;

b) generating outcome data configured to comprise

- at least one refractive error of the eye of the person, wherein the at least one refractive error of the eye of the person is determined by evaluating the input data, wherein the position of the entrance pupil of the eye of the person is considered when the at least one refractive error of the eye of the person is determined.

As used herein, the term "refractive value" refers to a characteristic chosen to counteract a refractive error as defined above in more detail. The characteristic may be a characteristic of the geometrical model of at least one spectacle lens or of the at least one spectacle lens. As used herein, the term "vision testing procedure" refers to a procedure in which the at least one refractive error of an eye of a person is determined. The at least one vision testing procedure may comprise the steps of the computer-implemented method for determining at least one refractive error of an eye of a person as described elsewhere herein.

For further details concerning the method for producing a geometrical model, a reference may be made to any aspect and/or any definition according to the present invention as disclosed elsewhere herein.

According to a further aspect, the present invention relates a method for producing at least one spectacle lens according to claim 11, wherein the at least one spectacle lens is manufactured by processing at least one lens blank, wherein the processing of the at least one lens blank is performed by using the produced geometrical model of the at least one spectacle lens as generated by a method for producing the geometrical model of the at least one spectacle lens for manufacturing of the at least one spectacle lens as defined in claim 10.

Referring to the computer-implemented aspects of the invention, one or more of the method steps or even all of the method steps of the method as disclosed herein may be performed by using a computer or a computer network. Thus, generally, any of the method steps including provision and/or manipulation of data may be performed by using the computer or the computer network. Generally, these method steps may include any of the method steps, typically except for method steps requiring manual work, such as certain aspects of performing the actual measurements.

Various embodiments may be conceived for implementing the methods according to the present invention. According to a first embodiment, all method steps may be performed by using a single processing device, such as a computer, especially a virtual reality headset, an augmented reality system, a desktop computer, a television set, smart glasses, or a mobile communication device, specifically a smartphone. In this embodiment, the single processing device may be configured to exclusively perform at least one computer program, in particular at least one line of computer program code configured to execute at least one algorithm, as used in at least one of the methods according to the present invention. Herein, the computer program as executed on the single processing device may comprise all instructions causing the computer to carry out at least one of the methods according to the present invention. Alternatively, or in addition, at least one method step may be performed by using at least one remote processing device, especially selected from at least one of a server or a cloud server, which is not located at the site of the person when executing the at least one method step. In this further embodiment, the computer program may comprise at least one remote portion to be executed by the at least one remote processing device to carry out the at least one method step. Further, the computer program may comprise at least one interface configured to forward to and/or receive data from the at least one remote portion of the computer program.

With respect to the prior art, the device exhibits the following advantages.

The invention provides a fast, easy, versatile, reliable, and accurate approach for determining at least one refractive error of an eye of a person.

By considering the entrance pupil of the eye of the person the determined at least one refractive error of the person may particularly be determined more exactly and/or accurate, compared to method in which the position of the entrance pupil of the eye of the person is not considered. Thereby, spectacle lenses produced by considering the at least one refractive error of the person that is determined in line with the presented invention, may correct any actual refractive error of the eye of the person more exactly and/or more accurate. Particularly as a subjective influence of the person on the individual refraction values may be considered due to the self-adjustment of the distance of the person performing the measurement by herself or himself.

Considering the entrance pupil of the eye of the person may provide a fast, easy, versatile, reliable, and accurate approach for determining at least one refractive error of an eye of a person. It may further provide a method for measuring a refractive error of at least one eye of a person, which does may not require the use of refractive correction glasses during the measurement process. It may further provide a sensor-based distance measurement in real time having a reduced error, particularly a reduced error that may typically effect the distance measurement, more particularly by considering corresponding meridians.

As used herein, the terms "have", "comprise" or "include" or any arbitrary grammatical variation thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

As further used herein, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in this way with other features of the invention.

Short description of the Figures

Further optional features and embodiments of the present invention are disclosed in more detail in the subsequent description. It is emphasized here that the scope of the invention is not restricted by the preferred embodiments.

In the Figures:

Figure 1   illustrates an exemplary determining device for determining at least one refractive error of an eye of a person;

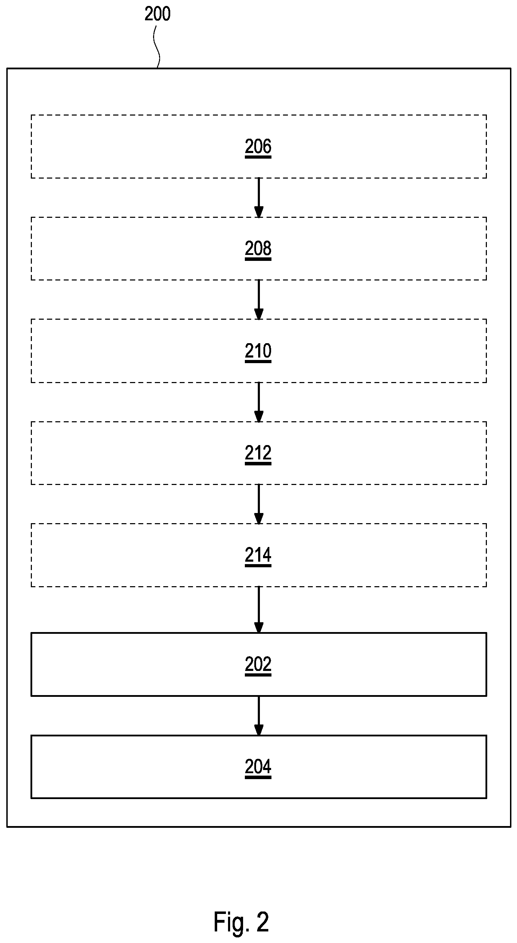

Figure 2   illustrates a computer-implemented method for determining at least one refractive error of an eye of a person;

Figure 3   illustrates a position of the entrance pupil of the eye of the person considered for determining at least one refractive error of an eye of a person;

Figure 4   illustrates an exemplary field device and an exemplary remote device; and

Figure 5   illustrates a method for producing a geometrical model and a method for producing at least one spectacle lens.

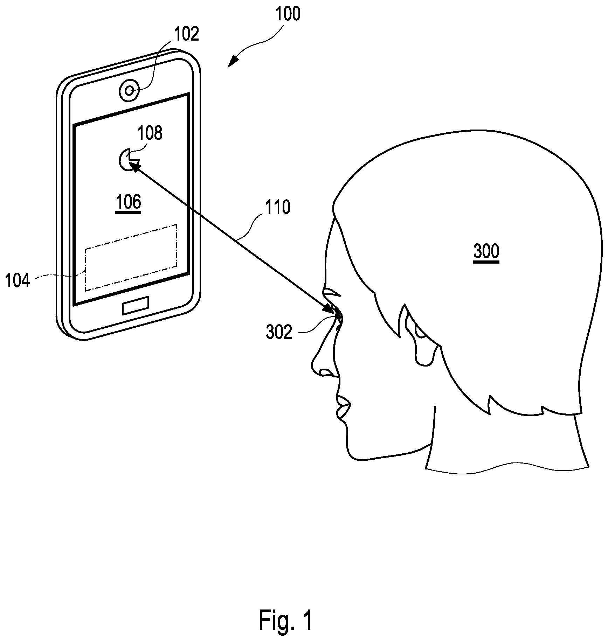

Figure 1 shows an exemplary determining device 100 for determining at least one refractive error of an eye 302 of a person 300. The determining device 100 is configured to carry out a computer-implemented method for determining at least one refractive error of an eye 302 of a person 300, as described in detail in the below. The refractive error of the eye 302 of the person 300 may be a value related to a spherical power, and/or a cylindrical power, and/or a cylinder axis, and/or an addition power.

The exemplary determining device 100 according to Fig. 1 is a smartphone. Alternatively, or in addition, the determining device 100 may be a smart watch and/or a desktop computer and/or a laptop and/or a tablet and/or a smart television.

For carrying out the computer-implemented method for determining at least one refractive error of an eye 302 of a person 300 the exemplary determining device 100 comprises a distance measuring unit 102 and a processing device 104. Further the exemplary determining device 100 comprises at least one screen 106 for displaying at least one visual stimulus 108. By using the distance measuring unit 102 at least one distance 110 between the eye 302 of the person 300 and the at least one visual stimulus 108 is recorded.

An exemplary distance measuring unit 102 comprised by the exemplary determining device 100 according to Fig. 1 is an image capturing unit, specifically the front camera of the smartphone. Alternatively, or in addition, the distance measuring unit 102 may be a sensor-based capturing unit, particularly an infra-red emitter and detector and/or a LiDAR module and/or a Bluetooth module. Further, alternatively or in addition, the distance measuring unit 102 may be an ultra-wideband unit, particularly comprising at least two communication devices, and/or an independent measurement device, particularly wherein the measurement may be performed by the person 300, specifically wherein the independent measurement device may be a ruler.

A computer program comprising instructions which, when the program is executed by a computer, cause the computer device to carry out the method for determining at least one refractive error of an eye 302 of a person 300, is exemplarily running on the determining device 100 according to Fig. 1.

The computer-implemented method 200 for determining at least one refractive error of an eye 302 of a person 300 is comprising the following steps:

a) generating input data 202 configured to comprise

- at least one distance 110 between the eye 302 of the person 300 and at least one visual stimulus 108 106 displayed to the eye 302 of the person 300, wherein the at least one distance 110 108 between the eye 302 of the person 300 is determined by

  o displaying to the eye 302 of the person 300 at least one visual stimulus 108 on at least one screen 106 while the distance 110 between the eye 302 of the person 300 and the at least one visual stimulus 108 displayed to the eye 302 of the person 300 is changed;
  o recording the at least one at least one distance 110 by using a distance measuring unit 102 at a point in time at which a visual perception of at least one spatial characteristic of the at least one visual stimulus 108 perceived by the person 300 changes;

- a position of an entrance pupil 304 of the eye 302 of the person 300;

b) generating outcome data 204 configured to comprise

- at least one refractive error of the eye 302 of the person 300, wherein the at least one refractive error of the eye 302 of the person 300 is determined by evaluating the input data, wherein the position of the entrance pupil 304 of the eye 302 of the person 300 is considered when the at least one refractive error of the eye 302 of the person 300 is determined.

The input data may comprise measured data, wherein specifically the at least one distance 110 between the eye 302 of the person 300 and the at least one visual stimulus 108 displayed to the eye 302 of the person 300 and/or the position of the entrance pupil 304 of the eye 302 of the person 300 is measured. Additionally, or alternatively, the person 300 may be a real person 300. The method may comprise actively determining the measured data.

The person 300 may be using an optical lens for the eye 302 of the person 300 while the input data is generated, particularly wherein the optical lens may have a known prescription. The at least one optical lens may be a spectacle lens, and/or a contact lens, and/or an intraocular lens.

The method may additionally comprise a requesting step 206, wherein the person 300 is requested to provide information related to the person 300 in the requesting step 206. The information related to the person 300 may comprise an age of the person 300, and/or an ethnicity of the person 300, and/or a gender of the person 300, and/or a location of the person 300 at which at least a part of the input data is generated, particularly at which the at least one distance 110 is measured, and/or a prescription of the at least one optical lens currently used by the person 300, and/or an indication of the eye 302 of the person 300 for which the at least one refractive error is determined, and/or a vertex distance 110 related to a spectacle lens for the eye 302 currently used by the person 300.

The at least one screen 106 may be comprised by a smartphone (as exemplarily depicted in Fig. 1), and/or a smart watch, and/or a desktop computer, and/or a laptop, and/or a tablet, and/or a smart television.

The distance measuring unit 102 may be an image capturing unit such as a camera, and/or a sensor-based capturing unit. The sensor-based capturing unit may be an infra-red emitter and detector, and/or a LiDAR module, and/or a Bluetooth module. Alternatively, or in addition, the distance measuring unit 102 may be an ultra-wideband unit, particularly comprising at least two communication devices, and/or an independent measurement device, particularly wherein the measurement is performed by the person 300, specifically wherein the independent measurement device is a ruler.

A type of the at least one visual stimulus 108 may be a Gabor Patch, and/or a grid, and/or a circle, and/or a point, and/or an optotype. The type of the optotype may be a Landolt C, and/or a Snellen E, and/or a Sloan letter, and/or an optometric image symbol, and/or a letter, and/or a text, and/or a number, and/or a natural picture, and/or a movie scene. The at least one visual stimulus 108 may be vanishing.

The method may comprise a selecting step 208, wherein the person 300 may be requested to choose at least one characteristic of the at least one visual stimulus 108. The at least one characteristic to choose may be a type of the at least one visual stimulus 108, specifically a type of the optotype, and/or a color of the at least one visual stimulus 108, and/or an orientation of the at least one visual stimulus 108, and/or a spatial frequency of the at least one of the visual stimulus 108, and/or a size of the at least one of the visual stimulus 108, and/or a contrast level of the at least one of the visual stimulus 108, and/or a polarization of the at least one of the visual stimulus 108, and/or a flicker frequency of the at least one of the visual stimulus 108, and/or a display time of the at least one of the visual stimulus 108, and/or a shape of the at least one of the visual stimulus 108.

A further eye 302 of the person 300 for which the at least one refractive error is not determined may be shielded from the at least one visual stimulus 108 displayed to the eye 302 of the person 300. The further eye 302 of the person 300 may be shielded by closing the further eye 302, and/or covering the further eye 302, specifically by using a cover, preferably a cardboard or a hand of the person 300 or of a further person 300.

The distance 110 between the eye 302 of the person 300 and the at least one visual stimulus 108 displayed to the eye 302 of the person 300 may be changed by moving the eye 302 of the person 300 towards the at least one screen 106, and/or moving the at least one screen 106 towards the eye 302 of the person 300, and/or changing the geometry of the field of view of the eye 302 of the person 300 between the eye 302 and the at least one visual stimulus 108 300, particularly by using at least one optical lens, and/or at least one mirror. Alternatively, or in addition, the distance 110 between the eye 302 of the person 300 and the at least one visual stimulus 108 displayed to the eye 302 of the person 300 may be changed by changing a virtual reality.

The distance 110 between the eye 302 of the person 300 and the at least one visual stimulus 108 displayed to the eye 302 of the person 300 may be changed by, particularly monotonously, decreasing the distance 110. The distance 110 between the eye 302 of the person 300 and the at least one visual stimulus 108 displayed to the eye 302 of the person 300 may be decreased from a first starting position.

The person 300 may be holding the at least one screen 106 in at least one hand and may be actively changing the distance 110 between the eye 302 of the person 300 and the at least one visual stimulus 108 displayed to the eye 302 of the person 300. The at least one screen 106 may be fixedly positioned, particularly on a stable ground, in front of the person 300 and the person 300 may be actively changing the distance 110 between the eye 302 of the person 300 and the at least one visual stimulus 108 displayed to the eye 302 of the person 300. The person 300 may be holding the at least one screen 106 in at least one hand, wherein at least one arm comprising the at least one hand is, particularly fully, extended at the first starting position.

The distance 110 between the eye 302 of the person 300 and the at least one visual stimulus 108 displayed to the eye 302 of the person 300 may be changed by, particularly monotonously, increasing the distance 110. The distance 110 between the eye 302 of the person 300 and the at least one visual stimulus 108 displayed to the eye 302 of the person 300 may be increased from a second starting position. The person 300 may be holding the at least one screen 106 in at least one hand, wherein the at least one screen 106 is positioned in close proximity of the eye 302 of the person 300 in the second starting position.

A plurality of visual stimuli may be displayed to the eye 302 of the person 300. At least two visual stimuli or each visual stimulus of the plurality of visual stimuli differ in a type, specifically a type of the optotype, and/or a color, and/or an orientation, and/or a spatial frequency, and/or a size, and/or a contrast level, and/or a polarization, and/or a flicker frequency, and/or a display time, and/or a shape. The at least two visual stimuli or each visual stimulus of the plurality of visual stimuli may have a different display time.

The at least one distance 110 may be measured at a point in time at which the visual perception of the at least one spatial characteristic of a fraction of the plurality of the visual stimuli perceived by the person 300 changes. The fraction may correspond to a maximum of 20%, of 40%, of 60% or of 80% of the plurality of visual stimuli.

A number of the plurality of the visual stimuli displayed to the eye 302 of the person 300, and/or a type of at least one visual stimulus 108 of the plurality of visual stimuli displayed to the eye 302 of the person 300, specifically a type of the optotype, and/or a color of at least one visual stimulus 108 of the plurality of visual stimuli displayed to the eye 302 of the person 300, and/or an orientation of at least one visual stimulus 108 of the plurality of visual stimuli displayed to the eye 302 of the person 300, and/or a spatial frequency of at least one visual stimulus 108 of the plurality of visual stimuli displayed to the eye 302 of the person 300, and/or a size of at least one visual stimulus 108 of the plurality of visual stimuli displayed to the eye 302 of the person 300, and/or a contrast level of at least one visual stimulus 108 of the plurality of visual stimuli displayed to the eye 302 of the person 300, and/or a polarization of at least one visual stimulus 108 of the plurality of visual stimuli displayed to the eye 302 of the person 300, and/or a flicker frequency of at least one visual stimulus 108 of the plurality of visual stimuli displayed to the eye 302 of the person 300, and/or a display time of at least one visual stimulus 108 of the plurality of visual stimuli displayed to the eye 302 of the person 300, may depend on a minimum angle of resolution MAR of the eye 302 of the person 300.

The visual perception of the at least one spatial characteristic of the at least one visual stimulus 108 perceived by the person 300 may change from blurred to sharp or from sharp to blurred at the point in time. It may be determined that the visual perception of the at least one spatial characteristic of the at least one visual stimulus 108 perceived by the person 300 changes and that the at least one distance 110 is to be recorded by an indication of the person 300, particularly wherein the indication is given by a visual signal, particularly a gesture, and/or an audio signal, particularly a voice input, specifically given by using a microphone, and/or a tactile signal, particularly by using a keypad, and/or a touch pad. Alternatively or in addition, it may be determined that the visual perception of the at least one spatial characteristic of the at least one visual stimulus 108 perceived by the person 300 changes and that the at least one distance 110 is to be recorded by an indication of a further person 300, particularly wherein the indication is given by a visual signal, particularly a gesture, and/or an audio signal, particularly a voice input, specifically given by using a microphone, and/or a tactile signal, particularly by using a keypad, and/or a touch pad. Further alternatively or in addition, it may be determined that the visual perception of the at least one spatial characteristic of the at least one visual stimulus 108 perceived by the person 300 changes and that the at least one distance 110 is to be recorded by a continuous distance 110 measurement and an automatic threshold estimation or calculation and/or a staircase method and/or forced choice method.

The at least one recorded distance 110 may corresponds to a near point distance 110, and/or a far point distance 110 of the eye 302 of the person 300.

It may be determined that the at least one recorded distance 110 corresponds to a near point distance 110 by an indication of the person 300, and/or an indication of the further person 300, and/or by considering if the distance 110 between the eye 302 of the person 300 and the at least one visual stimulus 108 is changed by increasing or decreasing, and/or by considering an age of the person 300, and/or by considering a table. It may be determined that the at least one recorded distance 110 corresponds to a far point distance 110 by an indication of the person 300, and/or an indication of the further person 300, and/or by considering if the distance 110 between the eye 302 of the person 300 and the at least one visual stimulus 108 is changed by increasing or decreasing, and/or by considering an age of the person 300, and/or by considering a table.

The at least one distance 110 between the eye 302 of the person 300 and the at least one visual stimulus 108 may be continuously measured. The size of the at least one visual stimulus 108 displayed to the eye 302 of the person 300 may be adjusted depending on the at least one distance 110 continuously measured between the eye 302 of the person 300 and the at least one visual stimulus 108. The size of the at least one visual stimulus 108 displayed to the eye 302 of the person 300 may be adjusted in a manner that the person 300 perceives the visual stimulus 108 in the same size independently from the at least one distance 110 continuously measured between the eye 302 of the person 300 and the at least one visual stimulus 108.

Step a) 202 of the method may define a measurement cycle. A plurality of measurement cycles may be performed. Step b) 204 of the method may define an evaluation cycle. A plurality of measurement cycles may be performed, wherein in each measurement cycle the at least one distance 110 is recorded, wherein each of the at least one recorded distance 110 may be considered in the evaluation cycle.

The method may further comprise an astigmatism determining step 210. In the astigmatism determining step 210 it may be determined if the eye 302 of the person 300 suffers from astigmatism by displaying a visual stimulus 108 configured for determining an astigmatism. The visual stimulus 108 configured for determining an astigmatism may be at least one line or a plurality of lines, particularly wherein the plurality of lines is grouped, specifically grouped in a manner according to which at least two lines of the plurality of lines are oriented in different directions. Alternatively or in addition, the visual stimulus 108 configured for determining an astigmatism may be a sun wheel or an astigmatism dial, and/or a Raubitschek figure, and/or a Gabor patch, and/or a grid, and/or a cross, and/or a Snellen E, and/or a Landolt C. Further, in the astigmatism determining step 210 an indication may be requested of the person 300 for testing if the eye 302 of the person 300 suffers from astigmatism. The astigmatism may be determined by using a forced choice principle.

The method may further comprise a cylinder axis determining step 212, wherein the cylinder axis may be determined by displaying a visual stimulus 108 configured for determining a cylinder axis. The visual stimulus 108 configured for determining a cylinder axis may be a sun wheel, and/or an astigmatism dial, and/or at least one line or a plurality of lines, particularly wherein the plurality of lines is grouped, specifically wherein the line are oriented in different directions, and/or a Raubitschek figure, and/or a Gabor patch, and/or a grid, and/or a cross, and/or a Snellen E, and/or a Landolt C. Further the cylinder axis may be determined by requesting an indication of the person 300 for determining the cylinder axis. Alternatively or in addition, the cylinder axis may be determined by considering a known cylinder axis value from a known prescription. The visual stimulus 108 may be oriented in a manner defined by the determined cylinder axis, particularly considering the meridians defined by the cylinder axis.

A distal far point distance 110, and/or a proximal far point distance 110, and/or a distal near point distance 110, and/or a proximal near point distance 110 may be recorded, particularly when the eye 302 of the person 300 suffers from astigmatism. When the eye 302 of the person 300 suffers from astigmatism, at least two measurement cycles may be performed, wherein in a first measurement cycle the visual stimulus 108 is oriented in a manner configured for a recording a distal far point distance 110 and/or a distal near point distance 110, and wherein in a second measurement cycle the visual stimulus 108 may be oriented in a manner configured for a recording of a proximal far point distance 110, and/or a proximal near point distance 110.

The eye 302 of the person 300 may suffer from astigmatism, so that at least three measurement cycles are performed, wherein in each measurement cycle a different meridian may be used, wherein a first meridian may be at 0°, wherein a second meridian may be at 60° and wherein a third meridian may be at 120°.

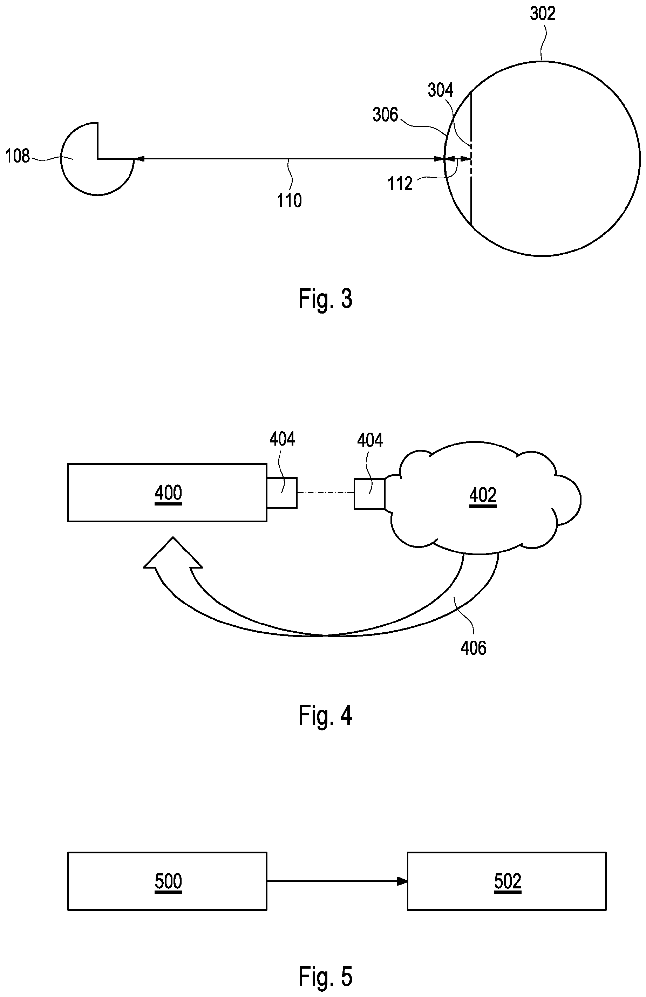

As illustrated in Fig. 3, considering the position of the entrance pupil 304 of the eye 302 of the person 300 may comprise considering the position of the entrance pupil 304 in relation to at least one further portion of the eye 302 of the person 300. The considered at least one further portion of the eye 302 of the person 300 may be the cornea 306 of the eye 302 of the person 300. According to the invention, considering the position of the entrance pupil 304 in relation to at least one further portion of the eye 302 of the person 300 comprises considering distance 112 between the entrance pupil 304, particularly a center of the entrance pupil 304, and the cornea 306. The distance 112 between the entrance pupil 304 and the cornea 306 is added to the distance 110 between the eye 302 of the person 300 and the at least one visual stimulus 108 for considering the position of the entrance pupil 304. The distance 112 between the entrance pupil 304 and the cornea 306 may be determined by considering an average radius of the cornea 306 of the eye 302 of the person 300, particularly by dividing the average radius of the cornea 306 of the eye 302 of the person 300 by factor of 2. At least one characteristic of the cornea 306 may be measured for considering the position of the entrance pupil 304. The at least one measured characteristic may be a curvature of the cornea 306, particularly average radius of the cornea 306.

The method may comprise a characteristic of the cornea 306 measuring step 214, wherein the characteristic of the cornea 306 may be measured by using an ophthalmometer or keratometer, particularly for determining a corneal topography; more particularly considering at least one reflection on the cornea; and/or a keratometry, particularly involving the use of a video, specifically a Placido method. Alternatively or in addition, the characteristic of the cornea 306 may be measured by using a smartphone-based video keratometry tomography; particularly by considering an optical coherence tomography, OCT; and/or a Scheimpflug principle.

The at least one characteristic of the cornea 306 may be measured before a first measurement; and/or during a measurement cycle; and/or after a measurement cycle; and/or after a final measurement cycle. The at least one characteristic may be measured multiple times.

For determining the refractive error of the eye 302 of the person 300 an age dependent accommodation amplitude AA or an age dependent near point distance 110, aNPD, may be considered, particularly for correcting the measured near point distance 110, NPD.

The age dependent accommodation amplitude AA in meter for a person 300 being 52 years old or being younger than 52 years old may be determined by using equation (1)

$$AA = 15.6 - \frac{1}{3} * \text{age}, \qquad (1)$$

wherein age is the age of the person 300.

The age dependent accommodation amplitude AA may have a value of 0 for a person 300 being older than 52. The age dependent accommodation amplitude AA may be determined from reading a table. The age dependent near point distance 110 aNPD may be determined from reading a table.

For determining the refractive error of the eye 302 of the person 300 a parameter K may be considered, wherein the parameter K depends on a first color of the visual stimulus 108 and a second color of the background on which the visual stimulus 108 is displayed.

The parameter K may depend on at least one spectrum emitted by the at least one screen 106 for displaying the background; and/or the visual stimulus 108. It may be determined that the eye 302 of the person 300 suffers from myopia by a far vision test, particularly a refraction test specifically an objective or subjective refraction test; and/or a duochrome test and/or considering a known prescription.

The spherical power SPH in diopter may be determined by using equation (2)

$$SPH = -\frac{1}{FPD + dEP}, \qquad (2)$$

FPD is the far point distance 110 and dEP is the distance 110 between the entrance pupil 304 and the cornea 306. The spherical power SPH in diopter may be determined by using equation (3)

$$SPH = -\frac{1}{FPD + dEP} + K, \qquad (3)$$

wherein FPD is the far point distance 110, dEP is the distance 110 between the entrance pupil 304 and the cornea 306 as well as K is a parameter dependent on the first color of the visual stimulus 108 and the second color of the background on which the visual stimulus 108 is displayed.

The eye 302 of the person 300 may further suffer from astigmatism, wherein for calculating the spherical power the far point distance 110 FPD is the distal far point distance 110, dFPD.

The cylindrical power CYL may be determined by using equation (4)

$$CYL = -\left(\frac{1}{pFPD} - \frac{1}{dFPD}\right), \tag{4}$$

wherein pFPD is the proximal far point distance 110 and dFPD is the distal far point distance 110. Alternatively or in addition, the cylindrical power CYL may be determined by using equation (5)

$$CYL = -\left(\frac{1}{pFPD+dEP} - \frac{1}{dFPD+dEP}\right), \tag{5}$$

wherein pFPD is the proximal far point distance 110, dFPD is the distal far point distance 110 and dEP is the distance 110 between the entrance pupil 304 and the cornea 306. Alternatively or in addition, the cylindrical power CYL may be determined by using equation (6)

$$CYL = -\left(\frac{1}{pFPD+dEP} - \frac{1}{dFPD+dEP}\right) + K, \tag{6}$$

wherein pFPD is the proximal far point distance 110, dFPD is the distal far point distance 110, dEP is the distance 110 between the entrance pupil 304 and the cornea 306 and K is the correction factor of the longitudinal chromatic aberration.

It may be determined that the eye 302 of the person 300 suffers from hyperopia by a near vision test, particularly a refraction test specifically an objective or subjective refraction test; and/or a duochrome test; and/or a reading Task; and/or a consideration of an age of the person 300 and/or considering a known prescription. The spherical power SPH in diopter may be determined by using equation (7)

$$SPH = -\frac{1}{NPD+dEP} + AA, \tag{7}$$

wherein NPD is the near point distance 110, dEP is the distance 110 between the entrance pupil 304 and the cornea 306 as well as AA is the age dependent accommodation amplitude. Alternatively or in addition, the spherical power SPH in diopter may be determined by using equation (8)

$$SPH = -\frac{1}{NPD+dEP} + AA + K, \tag{8}$$

wherein NPD is the near point distance 110, dEP is the distance 110 between the entrance pupil 304 and the cornea 306., AA is an age dependent accommodation amplitude and K is a parameter dependent on the first color of the visual stimulus 108 and the second color of the background on which the visual stimulus 108 is displayed.

Instead of the age dependent accommodation amplitude AA an age dependent near point distance 110 aNPD may considered. The age dependent near point distance 110 aNPD may be subtracted from the near point distance 110 NPD.

The eye 302 of the person 300 may further suffer from astigmatism, wherein for calculating the spherical power the near point distance 110 NPD may be the distal near point distance 110 dNPD.

The cylindrical power CYL may be determined by using equation (9)

$$CYL = -\left(\frac{1}{pNPD} - \frac{1}{dNPD}\right), \tag{9}$$

wherein pNPD is the proximal near point distance 110 and dNPD is the distal near point distance 110. Alternatively or in addition, the cylindrical power CYL may be determined by using equation (10)

$$CYL = -\left(\frac{1}{pFPD+dEP} - \frac{1}{dFPD+dEP}\right), \qquad (10)$$

wherein pFPD is the proximal far point distance 110, dFPD is the distal far point distance 110 and dEP is the distance 110 between the entrance pupil 304 and the cornea 306. Alternatively or in addition, the cylindrical power CYL may be determined by using equation (11)

$$CYL = -\left(\frac{1}{pFPD+dEP} - \frac{1}{dFPD+dEP}\right) + K, \qquad (11)$$

wherein pFPD is the proximal far point distance 110, dFPD is the distal far point distance 110, dEP is the distance 110 between the entrance pupil 304 and the cornea 306 and K is the correction factor of the longitudinal chromatic aberration.

For the eye 302 of the person 300 suffering from myopia and astigmatism a cylinder axis AXIS may be determined for

$$0° < \text{degree of pFPD} < 90°,$$

wherein pFPD is the proximal far point distance 110, by using equation (12)

$$AXIS = 90° - \text{degree of pFPD or}$$

$$AXIS = 270° - \text{degree of dFPD}, \qquad (12)$$

wherein dFPD is the distal far point distance 110.

For the eye 302 of the person 300 suffering from hyperopia and astigmatism a cylinder axis AXIS may be determined for

$$0° < \text{degree of pNPD} < 90°,$$

wherein pNPD is the proximal near point distance 110, by using equation (13)

$$AXIS = 90° - \text{degree of pNPD or}$$

$$AXIS = 270° - \text{degree of dNPD}, \qquad (13)$$

wherein dNPD is the distal near point distance 110.

According to Fig. 4, an exemplary field device 400 is shown. The field device 400 is for generating input data for determining at least one refractive error of an eye 302 of a person 300, wherein the field device 400 is configured to carry out a computer-implemented method for determining at least one refractive error of an eye 302 of a person 300, comprising the following steps:

a) generating input data configured to comprise

- at least one distance 110 between the eye 302 of the person 300 and at least one visual stimulus 108 displayed to the eye 302 of the person 300, wherein the at least one distance 110 between the eye 302 of the person 300 is determined by

o displaying to the eye 302 of the person 300 at least one visual stimulus 108 on at least one screen 106 while the distance 110 between the eye 302 of the person 300 and the at least one visual stimulus 108 displayed to the eye 302 of the person 300 is changed;

o recording the at least one at least one distance 110 by using a distance measuring unit 102 at a point in time at which a visual perception of at least one spatial characteristic of the at least one visual stimulus 108 perceived by the person 300 changes;

- a position of an entrance pupil 304 of the eye 302 of the person 300;

wherein the input data set is transmitted to a remote device 402 by using a connection interface 404 for determining at least one refractive error of the eye 302 of the person 300 by considering the position of an entrance pupil 304 of the eye 302 of the person 300.

The field device 400 may be a smartphone, and/or a smart watch, and/or a desktop computer, and/or a laptop, and/or a tablet, and/or a smart television.

Further according to Fig. 4, an exemplary remote device 402 is shown. The remote device 402 is for generating outcome data for determining at least one refractive error of an eye 302 of a person 300, wherein the device is configured to carry out a computer-implemented method for determining at least one refractive error of an eye 302 of a person 300, wherein the remote device 402 is receiving input data provided by a field device 400 by using a connection interface 404, wherein the method is comprising the following step:

b) generating outcome data by using a processing device 104 configured to comprise

- at least one refractive error of the eye 302 of the person 300, wherein the at least one refractive error of the eye 302 of the person 300 is determined by evaluating the input data, wherein a position of an entrance pupil 304 of the eye 302 of the person 300 is considered when the at least one refractive error of the eye 302 of the person 300 is determined.

The remote device 402 may be a server, and/or a cloud server. The connection interface 404 may be a network interface controller, and/or a transmitter.

Particularly the remote device 402 and/or the determining device 100, but not limited to these devices, may generate a data carrier signal 406 carrying the outcome data generated by a method as discussed in the above.

According to Fig. 5, a method for producing a geometrical model 500 of at least one spectacle lens for manufacturing of the at least one spectacle lens is illustrated, wherein producing the geometrical model comprises

- producing a geometrical model of at least one spectacle lens for at least one eye 302 of a person 300 by using data related to at least one refractive value; and
- determining the data related to the at least one refractive value by carrying out a computer-implemented method for determining at least one refractive error of an eye 302 of a person 300 in a vision testing procedure, wherein the vision testing procedure comprises at least one test cycle, preferably at least two subsequent test cycles, wherein the test cycle comprises at least the following steps:

a) generating input data 202 configured to comprise

- at least one distance 110 between the eye 302 of the person 300 and at least one visual stimulus 108 displayed to the eye 302 of the person 300, wherein the at least one distance 110 between the eye 302 of the person 300 is determined by

o displaying to the eye 302 of the person 300 at least one visual stimulus 108 on at least one screen 106 while the distance 110 between the eye 302 of the person 300 and the at least one visual stimulus 108 displayed to the eye 302 of the person 300 is changed;
o recording the at least one at least one distance 110 by using a distance measuring unit 102 at a point in time at which a visual perception of at least one spatial characteristic of the at least one visual stimulus 108 perceived by the person 300 changes;

- a position of an entrance pupil 304 of the eye 302 of the person 300;

b) generating outcome data 204 configured to comprise

- at least one refractive error of the eye 302 of the person 300, wherein the at least one refractive error of the eye 302 of the person 300 is determined by evaluating the input data, wherein the position of the entrance pupil 304 of the eye 302 of the person 300 is considered when the at least one refractive error of the eye 302 of the person 300 is determined.

Further according to Fig. 5, a method for producing at least one spectacle lens 502 is illustrated, wherein the at least one spectacle lens is manufactured by processing at least one lens blank by using the produced geometrical model of the at least one spectacle lens as discussed above.

List of Reference Signs

| | |
|---|---|
| 100 | determining device |
| 102 | distance measuring unit |
| 104 | processing device |
| 106 | screen |
| 108 | visual stimulus |
| 110 | distance |
| 112 | distance between the entrance pupil and the cornea of the eye of the person |
| 200 | computer-implemented method for determining at least one refractive error of an eye of a person |
| 202 | step a), generating input data |
| 204 | step b), generating outcome data |
| 206 | requesting step |
| 208 | selecting step |
| 210 | astigmatism determining step |
| 212 | cylinder axis determining step |
| 214 | characteristic of the cornea measuring step |
| 300 | person |
| 302 | eye |
| 304 | entrance pupil |
| 306 | cornea |
| 400 | field device |
| 402 | remote device |
| 404 | connection interface |
| 406 | data carrier signal |

**Claims**

1. A computer-implemented method (200) for determining at least one refractive error of an eye (302) of a person (300), the method comprising the following steps:

   a) generating input data (202) configured to comprise

      - at least one distance (110) between the eye (302) of the person (300) and at least one visual stimulus (108) displayed to the eye (302) of the person (300), wherein the at least one distance (110) between the eye (302) of the person (300) is determined by

         o displaying to the eye (302) of the person (300) at least one visual stimulus (108) on at least one screen (106) while the distance (110) between the eye (302) of the person (300) and the at least one visual stimulus (108) displayed to the eye (302) of the person (300) is changed;
         o recording the at least one distance (110) by using a distance measuring unit (102) at a point in time at which a visual perception of at least one spatial characteristic of the at least one visual stimulus (108) perceived by the person (300) changes;

      - a position of an entrance pupil (304) of the eye (302) of the person (300);

   b) generating outcome data (204) configured to comprise

      - at least one refractive error of the eye (302) of the person (300), wherein the at least one refractive error of the eye (302) of the person (300) is determined by evaluating the input data;

   **characterized in that**

the position of the entrance pupil (304) of the eye (302) of the person (300) is considered when the at least one refractive error of the eye (302) of the person (300) is determined, wherein considering the position of the entrance pupil (304) of the eye (302) of the person (300) comprises considering the position of the entrance pupil (304) in relation to at least one further portion of the eye (302) of the person (300), wherein considering the position of the entrance pupil (304) in relation to at least one further portion of the eye (302) of the person (300) comprises considering a distance (112) between the entrance pupil (304) and a cornea (306) of the eye (302) of the person (300), wherein the distance (112) between the entrance pupil (304) and the cornea (306) is added to the distance (110) between the eye (302) of the person (300) and the at least one visual stimulus (108) for considering the position of the entrance pupil (304).

2. The method (200) according to the preceding claim, wherein the refractive error of the eye (302) of the person (300) is at least one of a value related to:

- a spherical power;
- a cylindrical power;
- a cylinder axis; or
- an addition power.

3. The method (200) according to any one of the preceding claims, wherein the distance (110) between the eye (302) of the person (300) and the at least one visual stimulus (108) displayed to the eye (302) of the person (300) is changed by monotonously decreasing or monotonously increasing the distance (110).

4. The method (200) according to any one of the preceding claims, wherein the visual perception of at least one spatial characteristic of the at least one visual stimulus (108) perceived by the person (300) changes from blurred to sharp or from sharp to blurred at the point in time.

5. The method (200) according to any one of the preceding claims, wherein the at least one recorded distance (110) corresponds to at least one of:

- a near point distance (110); or
- a far point distance (110);

of the eye (302) of the person (300).

6. The method according to any one of the preceding claims, wherein the distance (112) between the entrance pupil (304) and the cornea (306) is determined by considering an average radius of the cornea (306) of the eye (302) of the person (300).

7. A computer program comprising instructions which, when the program is executed by a computer, cause the computer to carry out a computer-implemented method (200) for determining at least one refractive error of an eye (302) of a person (300), the method (200) comprising the following steps:

a) generating input data (202) configured to comprise

- at least one distance (110) between the eye (302) of the person (300) and at least one visual stimulus (108) displayed to the eye (302) of the person (300), wherein the at least one distance (110) between the eye (302) of the person (300) is determined by

o displaying to the eye (302) of the person (300) at least one visual stimulus (108) on at least one screen (106) while the distance (110) between the eye (302) of the person (300) and the at least one visual stimulus (108) displayed to the eye (302) of the person (300) is changed;
o recording the at least one distance (110) by using a distance measuring unit (102) at a point in time at which a visual perception of at least one spatial characteristic of the at least one visual stimulus (108) perceived by the person (300) changes;

- a position of an entrance pupil (304) of the eye (302) of the person (300);

b) generating outcome data (204) configured to comprise

- at least one refractive error of the eye (302) of the person (300), wherein the at least one refractive error of the eye (302) of the person (300) is determined by evaluating the input data,

**characterized in that**
the position of the entrance pupil (304) of the eye (302) of the person (300) is considered when the at least one refractive error of the eye (302) of the person (300) is determined, wherein considering the position of the entrance pupil (304) of the eye (302) of the person (300) comprises considering the position of the entrance pupil (304) in relation to at least one further portion of the eye (302) of the person (300), wherein considering the position of the entrance pupil (304) in relation to at least one further portion of the eye (302) of the person (300) comprises considering a distance (112) between the entrance pupil (304) and a cornea (306) of the eye (302) of the person (300), wherein the distance (112) between the entrance pupil (304) and the cornea (306) is added to the distance (110) between the eye (302) of the person (300) and the at least one visual stimulus (108) for considering the position of the entrance pupil (304).

8. A remote device (402) for generating outcome data for determining at least one refractive error of an eye (302) of a person (300), wherein the remote device (402) is configured to carry out a computer-implemented method (200) for determining at least one refractive error of an eye (302) of a person (300), wherein the remote device (402) is receiving input data provided by a field device (400) by using a connection interface (404), wherein the method is comprising the following step:

b) generating outcome data (204) by using a processing device (104) configured to comprise

- at least one refractive error of the eye (302) of the person (300), wherein the at least one refractive error of the eye (302) of the person (300) is determined by evaluating the input data;

**characterized in that**
a position of an entrance pupil (304) of the eye (302) of the person (300) is considered when the at least one refractive error of the eye (302) of the person (300) is determined, wherein considering the position of the entrance pupil (304) of the eye (302) of the person (300) comprises considering the position of the entrance pupil (304) in relation to at least one further portion of the eye (302) of the person (300), wherein considering the position of the entrance pupil (304) in relation to at least one further portion of the eye (302) of the person (300) comprises considering a distance (112) between the entrance pupil (304) and a cornea (306) of the eye (302) of the person (300), wherein the distance (112) between the entrance pupil (304) and the cornea (306) is added to the distance (110) between the eye (302) of the person (300) and the at least one visual stimulus (108) for considering the position of the entrance pupil (304).

9. A determining device (100) for determining at least one refractive error of an eye (302) of a person (300), wherein the device is configured to carry out a computer-implemented method for determining at least one refractive error of an eye (302) of a person (300), wherein the method is comprising the following steps:

a) generating input data (202) configured to comprise

- at least one distance (110) between the eye (302) of the person (300) and at least one visual stimulus (108) displayed to the eye (302) of the person (300), wherein the at least one distance (110) between the eye (302) of the person (300) is determined by

o displaying to the eye (302) of the person (300) at least one visual stimulus (108) on at least one screen (106) while the distance (110) between the eye (302) of the person (300) and the at least one visual stimulus (108) displayed to the eye (302) of the person (300) is changed;
o recording the at least one distance (110) by using a distance measuring unit (102) at a point in time at which a visual perception of at least one spatial characteristic of the at least one visual stimulus (108) perceived by the person (300) changes;

- a position of an entrance pupil (304) of the eye (302) of the person (300);

b) generating outcome data (204) by using a processing device (104) configured to comprise

- at least one refractive error of the eye (302) of the person (300), wherein the at least one refractive error of the eye (302) of the person (300) is determined by evaluating the input data;

**characterized in that**

the position of the entrance pupil (304) of the eye (302) of the person (300) is considered when the at least one refractive error of the eye (302) of the person (300) is determined, wherein considering the position of the entrance pupil (304) of the eye (302) of the person (300) comprises considering the position of the entrance pupil (304) in relation to at least one further portion of the eye (302) of the person (300), wherein considering the position of the entrance pupil (304) in relation to at least one further portion of the eye (302) of the person (300) comprises considering a distance (112) between the entrance pupil (304) and a cornea (306) of the eye (302) of the person (300), wherein the distance (112) between the entrance pupil (304) and the cornea (306) is added to the distance (110) between the eye (302) of the person (300) and the at least one visual stimulus (108) for considering the position of the entrance pupil (304).

10. A method for producing a geometrical model (500) of at least one spectacle lens for manufacturing of the at least one spectacle lens, wherein producing the geometrical model comprises

- producing a geometrical model of at least one spectacle lens for at least one eye (302) of a person (300) by using data related to at least one refractive value; and
- determining the data related to the at least one refractive value by carrying out a computer-implemented method (200) for determining at least one refractive error of an eye (302) of a person (300) in a vision testing procedure, wherein the vision testing procedure comprises at least one test cycle, wherein the test cycle comprises at least the following steps:

a) generating input data (202) configured to comprise

- at least one distance (110) between the eye (302) of the person (300) and at least one visual stimulus (108) displayed to the eye (302) of the person (300), wherein the at least one distance (110) between the eye (302) of the person (300) is determined by

o displaying to the eye (302) of the person (300) at least one visual stimulus (108) on at least one screen (106) while the distance (110) between the eye (302) of the person (300) and the at least one visual stimulus (108) displayed to the eye (302) of the person (300) is changed;
o recording the at least one distance (110) by using a distance measuring unit (102) at a point in time at which a visual perception of at least one spatial characteristic of the at least one visual stimulus (108) perceived by the person (300) changes;

- a position of an entrance pupil (304) of the eye (302) of the person (300);

b) generating outcome data (204) configured to comprise

- at least one refractive error of the eye (302) of the person (300), wherein the at least one refractive error of the eye (302) of the person (300) is determined by evaluating the input data;

**characterized in that**

the position of the entrance pupil (304) of the eye (302) of the person (300) is considered when the at least one refractive error of the eye (302) of the person (300) is determined, wherein considering the position of the entrance pupil (304) of the eye (302) of the person (300) comprises considering the position of the entrance pupil (304) in relation to at least one further portion of the eye (302) of the person (300), wherein considering the position of the entrance pupil (304) in relation to at least one further portion of the eye (302) of the person (300) comprises considering a distance (112) between the entrance pupil (304) and a cornea (306) of the eye (302) of the person (300), wherein the distance (112) between the entrance pupil (304) and the cornea (306) is added to the distance (110) between the eye (302) of the person (300) and the at least one visual stimulus (108) for considering the position of the entrance pupil (304).

11. A method for producing at least one spectacle lens, wherein the at least one spectacle lens is manufactured by processing at least one lens blank by using a produced geometrical model, wherein the method includes producing the geometrical model by performing the method for producing a geometrical model (500) of at least one spectacle lens for manufacturing of the at least one spectacle lens according to the preceding claim.

**Patentansprüche**

1. Computerimplementiertes Verfahren (200) zum Bestimmen mindestens eines Brechungsfehlers eines Auges (302) einer Person (300), das Verfahren die folgenden Schritte umfassend:

    a) Erzeugen von Eingabedaten (202), die konfiguriert sind, Folgendes zu umfassen

    - mindestens eine Distanz (110) zwischen dem Auge (302) der Person (300) und mindestens einem visuellen Stimulus (108), der dem Auge (302) der Person (300) angezeigt wird, wobei die mindestens eine Distanz (110) zwischen dem Auge (302) der Person (300) bestimmt wird durch

        o Anzeigen, dem Auge (302) der Person (300), mindestens eines visuellen Stimulus (108) auf mindestens einem Bildschirm (106), während die Distanz (110) zwischen dem Auge (302) der Person (300) und dem mindestens einem visuellen Stimulus (108), der dem Auge (302) der Person (300) angezeigt wird, verändert wird,
        o Aufzeichnen der mindestens einen Distanz (110) unter Verwendung einer Distanzmesseinheit (102) zu einem Zeitpunkt, zu dem sich eine visuelle Wahrnehmung mindestens eines räumlichen Charakteristikums des mindestens einen visuellen Stimulus (108), der von der Person (300) wahrgenommen wird, verändert;

    - eine Position einer Eintrittspupille (304) des Auges (302) der Person (300);

    b) Erzeugen von Ergebnisdaten (204), die konfiguriert sind, Folgendes zu umfassen

        - mindestens einen Brechungsfehler des Auges (302) der Person (300), wobei der mindestens eine Brechungsfehler des Auges (302) der Person (300) durch Auswerten der Eingabedaten bestimmt wird;

    **dadurch gekennzeichnet, dass**
    die Position der Eintrittspupille (304) des Auges (302) der Person (300) berücksichtigt wird, wenn der mindestens eine Brechungsfehler des Auges (302) der Person (300) bestimmt wird, wobei die Berücksichtigung der Position der Eintrittspupille (304) des Auges (302) der Person (300) die Berücksichtigung der Position der Eintrittspupille (304) in Bezug auf mindestens einen weiteren Abschnitt des Auges (302) der Person (300) umfasst, wobei die Berücksichtigung der Position der Eintrittspupille (304) in Bezug auf mindestens einen weiteren Abschnitt des Auges (302) der Person (300) die Berücksichtigung einer Distanz (112) zwischen der Eintrittspupille (304) und einer Hornhaut (306) des Auges (302) der Person (300) umfasst, wobei die Distanz (112) zwischen der Eintrittspupille (304) und der Hornhaut (3006) zu der Distanz (110) zwischen dem Auge (302) der Person (300) und dem mindestens einen visuellen Stimulus (108) zur Berücksichtigung der Position der Eintrittspupille (304) addiert wird.

2. Verfahren (200) nach dem vorhergehenden Anspruch, wobei der Brechungsfehler des Auges (302) der Person (300) mindestens einer eines Werts ist, der sich auf Folgendes bezieht:

    - eine sphärische Brechkraft;
    - eine zylindrische Brechkraft;
    - eine Zylinderachse; oder
    - eine Additionsbrechkraft.

3. Verfahren (200) nach einem der vorhergehenden Ansprüche, wobei die Distanz (110) zwischen dem Auge (302) der Person (300) und dem mindestens einen visuellen Stimulus (108), der dem Auge (302) der Person (300) angezeigt wird, durch monotones Verringern oder monotones Vergrößern der Distanz (110) verändert wird.

4. Verfahren (200) nach einem der vorhergehenden Ansprüche, wobei die visuelle Wahrnehmung mindestens eines räumlichen Charakteristikums des mindestens einen visuellen Stimulus (108), der von der Person (300) wahrgenommen wird, sich von verschwommen zu scharf oder von scharf zu verschwommen zu dem Zeitpunkt verändert.

5. Verfahren (200) nach einem der vorhergehenden Ansprüche, wobei die mindestens eine aufgezeichnete Distanz (110) mit mindestens einem von Folgendem korrespondiert

    - eine Nahpunktdistanz (110); oder

- eine Fernpunktdistanz (110);

des Auges (302) der Person (300).

6. Verfahren nach einem der vorhergehenden Ansprüche, wobei die Distanz (112) zwischen der Eintrittspupille (304) und der Hornhaut (306) durch Berücksichtigung eines Durchschnittsradius der Hornhaut (306) des Auges (302) der Person (300) bestimmt wird.

7. Computerprogramm, umfassend Anweisungen, die, wenn das Programm von einem Computer ausgeführt wird, den Computer veranlassen, ein computerimplementiertes Verfahren (200) zum Bestimmen mindestens eines Brechungsfehlers eines Auges (302) einer Person (300) auszuführen, das Verfahren (200) die folgenden Schritte umfassend:

a) Erzeugen von Eingabedaten (202), die konfiguriert sind, Folgendes zu umfassen

- mindestens eine Distanz (110) zwischen dem Auge (302) der Person (300) und mindestens einem visuellen Stimulus (108), der dem Auge (302) der Person (300) angezeigt wird, wobei die mindestens eine Distanz (110) zwischen dem Auge (302) der Person (300) bestimmt wird durch

o Anzeigen, dem Auge (302) der Person (300), mindestens eines visuellen Stimulus (108) auf mindestens einem Bildschirm (106), während die Distanz (110) zwischen dem Auge (302) der Person (300) und dem mindestens einem visuellen Stimulus (108), der dem Auge (302) der Person (300) angezeigt wird, verändert wird,
o Aufzeichnen der mindestens einen Distanz (110) unter Verwendung einer Distanzmesseinheit (102) zu einem Zeitpunkt, zu dem sich eine visuelle Wahrnehmung mindestens eines räumlichen Charakteristikums des mindestens einen visuellen Stimulus (108), der von der Person (300) wahrgenommen wird, verändert;

- eine Position einer Eintrittspupille (304) des Auges (302) der Person (300);

b) Erzeugen von Ergebnisdaten (204), die konfiguriert sind, Folgendes zu umfassen

- mindestens einen Brechungsfehler des Auges (302) der Person (300), wobei der mindestens eine Brechungsfehler des Auges (302) der Person (300) durch Auswerten der Eingabedaten bestimmt wird,

**dadurch gekennzeichnet, dass**
die Position der Eintrittspupille (304) des Auges (302) der Person (300) berücksichtigt wird, wenn der mindestens eine Brechungsfehler des Auges (302) der Person (300) bestimmt wird, wobei die Berücksichtigung der Position der Eintrittspupille (304) des Auges (302) der Person (300) die Berücksichtigung der Position der Eintrittspupille (304) in Bezug auf mindestens einen weiteren Abschnitt des Auges (302) der Person (300) umfasst, wobei die Berücksichtigung der Position der Eintrittspupille (304) in Bezug auf mindestens einen weiteren Abschnitt des Auges (302) der Person (300) die Berücksichtigung einer Distanz (112) zwischen der Eintrittspupille (304) und einer Hornhaut (306) des Auges (302) der Person (300) umfasst, wobei die Distanz (112) zwischen der Eintrittspupille (304) und der Hornhaut (3006) zu der Distanz (110) zwischen dem Auge (302) der Person (300) und dem mindestens einen visuellen Stimulus (108) zur Berücksichtigung der Position der Eintrittspupille (304) addiert wird.

8. Entfernte Vorrichtung (402) zum Erzeugen von Ergebnisdaten zum Bestimmen mindestens eines Brechungsfehlers eines Auges (302) einer Person (300), wobei die entfernte Vorrichtung (402) konfiguriert ist, ein computerimplementiertes Verfahren (200) zum Bestimmen mindestens eines Brechungsfehlers eines Auges (302) einer Person (300) auszuführen, wobei die entfernte Vorrichtung (402) Eingabedaten, die von einer Feldvorrichtung (400) bereitgestellt werden, unter Verwendung einer Verbindungsschnittstelle (404) empfängt, wobei das Verfahren den folgenden Schritt umfasst:
b) Erzeugen von Ergebnisdaten (204) unter Verwendung einer Verarbeitungsvorrichtung (104), die konfiguriert sind, Folgendes zu umfassen

- mindestens einen Brechungsfehler des Auges (302) der Person (300), wobei der mindestens eine Brechungsfehler des Auges (302) der Person (300) durch Auswerten der Eingabedaten bestimmt wird;

**dadurch gekennzeichnet, dass**

eine Position einer Eintrittspupille (304) des Auges (302) der Person (300) berücksichtigt wird, wenn der mindestens eine Brechungsfehler des Auges (302) der Person (300) bestimmt wird, wobei die Berücksichtigung der Position der Eintrittspupille (304) des Auges (302) der Person (300) die Berücksichtigung der Position der Eintrittspupille (304) in Bezug auf mindestens einen weiteren Abschnitt des Auges (302) der Person (300) umfasst, wobei die Berücksichtigung der Position der Eintrittspupille (304) in Bezug auf mindestens einen weiteren Abschnitt des Auges (302) der Person (300) die Berücksichtigung einer Distanz (112) zwischen der Eintrittspupille (304) und einer Hornhaut (300) des Auges (302) der Person (300) umfasst, wobei die Distanz (112) zwischen der Eintrittspupille (304) und der Hornhaut (306) zu der Distanz (110) zwischen dem Auge (302) der Person (300) und dem mindestens einen visuellen Stimulus (108) zur Berücksichtigung der Position der Eintrittspupille (304) addiert wird.

9. Bestimmungsvorrichtung (100) zum Bestimmen mindestens eines Brechungsfehlers eines Auges (302) einer Person (300), wobei die Vorrichtung konfiguriert ist, ein computerimplementiertes Verfahren zum Bestimmen mindestens eines Brechungsfehlers eines Auges (302) einer Person (300) auszuführen, wobei das Verfahren die folgenden Schritte umfasst:

   a) Erzeugen von Eingabedaten (202), die konfiguriert sind, Folgendes zu umfassen

   - mindestens eine Distanz (110) zwischen dem Auge (302) der Person (300) und mindestens einem visuellen Stimulus (108), der dem Auge (302) der Person (300) angezeigt wird, wobei die mindestens eine Distanz (110) zwischen dem Auge (302) der Person (300) bestimmt wird durch

      o Anzeigen, dem Auge (302) der Person (300), mindestens eines visuellen Stimulus (108) auf mindestens einem Bildschirm (106), während die Distanz (110) zwischen dem Auge (302) der Person (300) und dem mindestens einen visuellen Stimulus (108), der dem Auge (302) der Person (300) angezeigt wird, verändert wird,
      o Aufzeichnen der mindestens einen Distanz (110) unter Verwendung einer Distanzmesseinheit (102) zu einem Zeitpunkt, zu dem sich eine visuelle Wahrnehmung mindestens eines räumlichen Charakteristikums des mindestens einen visuellen Stimulus (108), der von der Person (300) wahrgenommen wird, verändert;

   - eine Position einer Eintrittspupille (304) des Auges (302) der Person (300);

   b) Erzeugen von Ergebnisdaten (204) unter Verwendung einer Verarbeitungsvorrichtung (104), die konfiguriert sind, Folgendes zu umfassen

   - mindestens einen Brechungsfehler des Auges (302) der Person (300), wobei der mindestens eine Brechungsfehler des Auges (302) der Person (300) durch Auswerten der Eingabedaten bestimmt wird;

   **dadurch gekennzeichnet, dass**
   die Position der Eintrittspupille (304) des Auges (302) der Person (300) berücksichtigt wird, wenn der mindestens eine Brechungsfehler des Auges (302) der Person (300) bestimmt wird, wobei die Berücksichtigung der Position der Eintrittspupille (304) des Auges (302) der Person (300) die Berücksichtigung der Position der Eintrittspupille (304) in Bezug auf mindestens einen weiteren Abschnitt des Auges (302) der Person (300) umfasst, wobei die Berücksichtigung der Position der Eintrittspupille (304) in Bezug auf mindestens einen weiteren Abschnitt des Auges (302) der Person (300) die Berücksichtigung einer Distanz (112) zwischen der Eintrittspupille (304) und einer Hornhaut (306) des Auges (302) der Person (300) umfasst, wobei die Distanz (112) zwischen der Eintrittspupille (304) und der Hornhaut (3006) zu der Distanz (110) zwischen dem Auge (302) der Person (300) und dem mindestens einen visuellen Stimulus (108) zur Berücksichtigung der Position der Eintrittspupille (304) addiert wird.

10. Verfahren zum Produzieren eines geometrischen Modells (500) mindestens einer Brillenglaslinse zum Herstellen der mindestens einen Brillenglaslinse, wobei das Produzieren des geometrischen Modells umfasst

   - Produzieren eines geometrischen Modells mindestens einer Brillenglaslinse für mindestens ein Auge (302) einer Person (300) unter Verwendung von Daten, die zu mindestens einem Brechungswert in Beziehung stehen; und
   - Bestimmen der Daten, die zu dem mindestens einen Brechungswert in Beziehung stehen, durch Ausführen eines computerimplementierten Verfahrens (200) zum Bestimmen mindestens eines Brechungsfehlers eines Auges (302) einer Person (300) in einer Sehtestprozedur, wobei die Sehtestprozedur mindestens einen Testzyklus umfasst, wobei der Testzyklus mindestens die folgenden Schritte umfasst:

a) Erzeugen von Eingabedaten (202), die konfiguriert sind, Folgendes zu umfassen

- mindestens eine Distanz (110) zwischen dem Auge (302) der Person (300) und mindestens einem visuellen Stimulus (108), der dem Auge (302) der Person (300) angezeigt wird, wobei die mindestens eine Distanz (110) zwischen dem Auge (302) der Person (300) bestimmt wird durch

o Anzeigen, dem Auge (302) der Person (300), mindestens eines visuellen Stimulus (108) auf mindestens einem Bildschirm (106), während die Distanz (110) zwischen dem Auge (302) der Person (300) und dem mindestens einen visuellen Stimulus (108), der dem Auge (302) der Person (300) angezeigt wird, verändert wird,
o Aufzeichnen der mindestens einen Distanz (110) unter Verwendung einer Distanzmesseinheit (102) zu einem Zeitpunkt, zu dem sich eine visuelle Wahrnehmung mindestens eines räumlichen Charakteristikums des mindestens einen visuellen Stimulus (108), der von der Person (300) wahrgenommen wird, verändert;

- eine Position einer Eintrittspupille (304) des Auges (302) der Person (300);

b) Erzeugen von Ergebnisdaten (204), die konfiguriert sind, Folgendes zu umfassen

- mindestens einen Brechungsfehler des Auges (302) der Person (300), wobei der mindestens eine Brechungsfehler des Auges (302) der Person (300) durch Auswerten der Eingabedaten bestimmt wird;

**dadurch gekennzeichnet, dass**
die Position der Eintrittspupille (304) des Auges (302) der Person (300) berücksichtigt wird, wenn der mindestens eine Brechungsfehler des Auges (302) der Person (300) bestimmt wird, wobei die Berücksichtigung der Position der Eintrittspupille (304) des Auges (302) der Person (300) die Berücksichtigung der Position der Eintrittspupille (304) in Bezug auf mindestens einen weiteren Abschnitt des Auges (302) der Person (300) umfasst, wobei die Berücksichtigung der Position der Eintrittspupille (304) in Bezug auf mindestens einen weiteren Abschnitt des Auges (302) der Person (300) die Berücksichtigung einer Distanz (112) zwischen der Eintrittspupille (304) und einer Hornhaut (306) des Auges (302) der Person (300) umfasst, wobei die Distanz (112) zwischen der Eintrittspupille (304) und der Hornhaut (306) zu der Distanz (110) zwischen dem Auge (302) der Person (300) und dem mindestens einen visuellen Stimulus (108) zur Berücksichtigung der Position der Eintrittspupille (304) addiert wird.

11. Verfahren zum Produzieren mindestens einer Brillenglaslinse, wobei die mindestens eine Brillenglaslinse durch Verarbeiten mindestens eines Linsenrohlings unter Verwendung eines produzierten geometrischen Modells hergestellt wird, wobei das Verfahren das Produzieren des geometrischen Modells durch Durchführen des Verfahrens zum Produzieren eines geometrischen Modells (500) mindestens einer Brillenglaslinse zum Herstellen der mindestens einen Brillenglaslinse nach dem vorhergehenden Anspruch enthält.

**Revendications**

1. Procédé mis en œuvre par ordinateur (200) pour déterminer au moins une erreur de réfraction d'un œil (302) d'une personne (300), le procédé comprenant les étapes suivantes :

a) la génération de données d'entrée (202) configurées pour comprendre

- au moins une distance (110) entre l'œil (302) de la personne (300) et au moins un stimulus visuel (108) affiché sur l'œil (302) de la personne (300), l'au moins une distance (110) entre l'œil (302) de la personne (300) étant déterminée par

o l'affichage sur l'œil (302) de la personne (300) d'au moins un stimulus visuel (108) sur au moins un écran (106) pendant que la distance (110) entre l'œil (302) de la personne (300) et l'au moins un stimulus visuel (108) affiché sur l'œil (302) de la personne (300) est modifiée ;
o l'enregistrement de l'au moins une distance (110) en utilisant une unité de mesure de distance (102) à un point temporel où une perception visuelle d'au moins une caractéristique spatiale de l'au moins un stimulus visuel (108) perçu par la personne (300) évolue ;

- une position d'une pupille d'entrée (304) de l'œil (302) de la personne (300) ;

b) la génération de données de résultat (204) configurées pour comprendre

- au moins une erreur de réfraction de l'œil (302) de la personne (300), l'au moins une erreur de réfraction de l'œil (302) de la personne (300) étant déterminée en évaluant les données d'entrée ;

**caractérisé en ce que**
la position de la pupille d'entrée (304) de l'œil (302) de la personne (300) est considérée quand l'au moins une erreur de réfraction de l'œil (302) de la personne (300) est déterminée, la considération de la position de la pupille d'entrée (304) de l'œil (302) de la personne (300) comprenant la considération de la position de la pupille d'entrée (304) par rapport à au moins une autre partie de l'œil (302) de la personne (300), la considération de la position de la pupille d'entrée (304) par rapport à au moins une autre partie de l'œil (302) de la personne (300) comprenant la considération d'une distance (112) entre la pupille d'entrée (304) et une cornée (306) de l'œil (302) de la personne (300), la distance (112) entre la pupille d'entrée (304) et la cornée (306) étant ajoutée à la distance (110) entre l'œil (302) de la personne (300) et l'au moins un stimulus visuel (108) pour considérer la position de la pupille d'entrée (304).

2. Procédé (200) selon la revendication précédente, l'erreur de réfraction de l'œil (302) de la personne (300) étant au moins l'une d'une valeur liée à :

- une puissance sphérique ;
- une puissance cylindrique ;
- un axe de cylindre ; ou
- une puissance d'addition.

3. Procédé (200) selon l'une quelconque des revendications précédentes, la distance (110) entre l'œil (302) de la personne (300) et l'au moins un stimulus visuel (108) affiché sur l'œil (302) de la personne (300) étant modifiée en diminuant de façon monotone ou en augmentant de façon monotone la distance (110).

4. Procédé (200) selon l'une quelconque des revendications précédentes, la perception visuelle d'au moins une caractéristique spatiale de l'au moins un stimulus visuel (108) perçu par la personne (300) passant de flou à net ou de net à flou à flou au point temporel.

5. Procédé (200) selon l'une quelconque des revendications précédentes, l'au moins une distance enregistrée (110) correspondant à au moins l'un des éléments suivants :

- une distance de point proche (110) ; ou
- une distance de point éloigné (110) ;

de l'œil (302) de la personne (300).

6. Procédé selon l'une quelconque des revendications précédentes, la distance (112) entre la pupille d'entrée (304) et la cornée (306) étant déterminée en considérant un rayon moyen de la cornée (306) de l'œil (302) de la personne (300).

7. Programme informatique comprenant des instructions qui, lorsque le programme est exécuté par un ordinateur, amènent l'ordinateur à réaliser un procédé mis en œuvre par ordinateur (200) pour déterminer au moins une erreur de réfraction d'un œil (302) d'une personne (300), le procédé (200) comprenant les étapes suivantes :

a) la génération de données d'entrée (202) configurées pour comprendre

- au moins une distance (110) entre l'œil (302) de la personne (300) et au moins un stimulus visuel (108) affiché sur l'œil (302) de la personne (300), l'au moins une distance (110) entre l'œil (302) de la personne (300) étant déterminée par

o l'affichage sur l'œil (302) de la personne (300) d'au moins un stimulus visuel (108) sur au moins un écran (106) pendant que la distance (110) entre l'œil (302) de la personne (300) et l'au moins un stimulus visuel (108) affiché sur l'œil (302) de la personne (300) est modifiée ;

o l'enregistrement de l'au moins une distance (110) en utilisant une unité de mesure de distance (102) à un point temporel où une perception visuelle d'au moins une caractéristique spatiale de l'au moins un stimulus visuel (108) perçu par la personne (300) évolue ;

- une position d'une pupille d'entrée (304) de l'œil (302) de la personne (300) ;

b) la génération de données de résultat (204) configurées pour comprendre

- au moins une erreur de réfraction de l'œil (302) de la personne (300), l'au moins une erreur de réfraction de l'œil (302) de la personne (300) étant déterminée en évaluant les données d'entrée,

**caractérisé en ce que**
la position de la pupille d'entrée (304) de l'œil (302) de la personne (300) est considérée quand l'au moins une erreur de réfraction de l'œil (302) de la personne (300) est déterminée, la considération de la position de la pupille d'entrée (304) de l'œil (302) de la personne (300) comprenant la considération de la position de la pupille d'entrée (304) par rapport à au moins une autre partie de l'œil (302) de la personne (300), la considération de la position de la pupille d'entrée (304) par rapport à au moins une autre partie de l'œil (302) de la personne (300) comprenant la considération d'une distance (112) entre la pupille d'entrée (304) et une cornée (306) de l'œil (302) de la personne (300), la distance (112) entre la pupille d'entrée (304) et la cornée (306) étant ajoutée à la distance (110) entre l'œil (302) de la personne (300) et l'au moins un stimulus visuel (108) pour considérer la position de la pupille d'entrée (304).

8. Dispositif distant (402) pour générer des données de résultat pour déterminer au moins une erreur de réfraction d'un œil (302) d'une personne (300), le dispositif distant (402) étant configuré pour réaliser un procédé mis en œuvre par ordinateur (200) pour déterminer au moins une erreur de réfraction d'un œil (302) d'une personne (300), le dispositif distant (402) recevant des données d'entrée fournies par un dispositif de terrain (400) en utilisant une interface de connexion (404), le procédé comprenant l'étape suivante :
b) la génération de données de résultat (204) à l'aide d'un dispositif de traitement (104) configuré pour comprendre

- au moins une erreur de réfraction de l'œil (302) de la personne (300), l'au moins une erreur de réfraction de l'œil (302) de la personne (300) étant déterminée en évaluant les données d'entrée ;

**caractérisé en ce que**
une position d'une pupille d'entrée (304) de l'œil (302) de la personne (300) est considérée quand l'au moins une erreur de réfraction de l'œil (302) de la personne (300) est déterminée, la considération de la position de la pupille d'entrée (304) de l'œil (302) de la personne (300) comprenant la considération de la position de la pupille d'entrée (304) par rapport à au moins une autre partie de l'œil (302) de la personne (300), la considération de la position de la pupille d'entrée (304) par rapport à au moins une autre partie de l'œil (302) de la personne (300) comprenant la considération d'une distance (112) entre la pupille d'entrée (304) et une cornée (306) de l'œil (302) de la personne (300), la distance (112) entre la pupille d'entrée (304) et la cornée (306) étant ajoutée à la distance (110) entre l'œil (302) de la personne (300) et l'au moins un stimulus visuel (108) pour considérer la position de la pupille d'entrée (304).

9. Dispositif de détermination (100) pour déterminer au moins une erreur de réfraction d'un œil (302) d'une personne (300), le dispositif étant configuré pour réaliser un procédé mis en œuvre par ordinateur de détermination d'au moins une erreur de réfraction d'un œil (302) d'une personne (300), le procédé comprenant les étapes suivantes :

a) la génération de données d'entrée (202) configurées pour comprendre

- au moins une distance (110) entre l'œil (302) de la personne (300) et au moins un stimulus visuel (108) affiché sur l'œil (302) de la personne (300), l'au moins une distance (110) entre l'œil (302) de la personne (300) étant déterminée par

o l'affichage sur l'œil (302) de la personne (300) d'au moins un stimulus visuel (108) sur au moins un écran (106) pendant que la distance (110) entre l'œil (302) de la personne (300) et l'au moins un stimulus visuel (108) affiché sur l'œil (302) de la personne (300) est modifiée ;
o l'enregistrement de l'au moins une distance (110) en utilisant une unité de mesure de distance (102) à un point temporel où une perception visuelle d'au moins une caractéristique spatiale de l'au moins un stimulus visuel (108) perçu par la personne (300) évolue ;

- une position d'une pupille d'entrée (304) de l'œil (302) de la personne (300) ;

b) la génération de données de résultat (204) à l'aide d'un dispositif de traitement (104) configuré pour comprendre

- au moins une erreur de réfraction de l'œil (302) de la personne (300), l'au moins une erreur de réfraction de l'œil (302) de la personne (300) étant déterminée en évaluant les données d'entrée ;

**caractérisé en ce que**
la position de la pupille d'entrée (304) de l'œil (302) de la personne (300) est considérée quand l'au moins une erreur de réfraction de l'œil (302) de la personne (300) est déterminée, la considération de la position de la pupille d'entrée (304) de l'œil (302) de la personne (300) comprenant la considération de la position de la pupille d'entrée (304) par rapport à au moins une autre partie de l'œil (302) de la personne (300), la considération de la position de la pupille d'entrée (304) par rapport à au moins une autre partie de l'œil (302) de la personne (300) comprenant la considération d'une distance (112) entre la pupille d'entrée (304) et une cornée (306) de l'œil (302) de la personne (300), la distance (112) entre la pupille d'entrée (304) et la cornée (306) étant ajoutée à la distance (110) entre l'œil (302) de la personne (300) et l'au moins un stimulus visuel (108) pour considérer la position de la pupille d'entrée (304).

10. Procédé de production d'un modèle géométrique (500) d'au moins un verre de lunettes pour la fabrication d'au moins un verre de lunettes, la production du modèle géométrique comprenant

- la production d'un modèle géométrique d'au moins un verre de lunettes pour au moins un œil (302) d'une personne (300) en utilisant des données relatives à au moins une valeur de réfraction ; et
- la détermination des données liées à l'au moins une valeur de réfraction en réalisant un procédé (200) mis en œuvre par ordinateur pour déterminer au moins une erreur de réfraction d'un œil (302) d'une personne (300) lors d'une procédure de test visuel, le procédé de test visuel comprenant au moins un cycle de test, le cycle de test comprenant au moins les étapes suivantes :

a) la génération de données d'entrée (202) configurées pour comprendre

- au moins une distance (110) entre l'œil (302) de la personne (300) et au moins un stimulus visuel (108) affiché sur l'œil (302) de la personne (300), l'au moins une distance (110) entre l'œil (302) de la personne (300) étant déterminée par

o l'affichage sur l'œil (302) de la personne (300) d'au moins un stimulus visuel (108) sur au moins un écran (106) pendant que la distance (110) entre l'œil (302) de la personne (300) et l'au moins un stimulus visuel (108) affiché sur l'œil (302) de la personne (300) est modifiée ;
o l'enregistrement de l'au moins une distance (110) en utilisant une unité de mesure de distance (102) à un point temporel où une perception visuelle d'au moins une caractéristique spatiale de l'au moins un stimulus visuel (108) perçu par la personne (300) évolue ;

- une position d'une pupille d'entrée (304) de l'œil (302) de la personne (300) ;

b) la génération de données de résultat (204) configurées pour comprendre

- au moins une erreur de réfraction de l'œil (302) de la personne (300), l'au moins une erreur de réfraction de l'œil (302) de la personne (300) étant déterminée en évaluant les données d'entrée ;

**caractérisé en ce que**
la position de la pupille d'entrée (304) de l'œil (302) de la personne (300) est considérée quand l'au moins une erreur de réfraction de l'œil (302) de la personne (300) est déterminée, la considération de la position de la pupille d'entrée (304) de l'œil (302) de la personne (300) comprenant la considération de la position de la pupille d'entrée (304) par rapport à au moins une autre partie de l'œil (302) de la personne (300), la considération de la position de la pupille d'entrée (304) par rapport à au moins une autre partie de l'œil (302) de la personne (300) comprenant la considération d'une distance (112) entre la pupille d'entrée (304) et une cornée (306) de l'œil (302) de la personne (300), la distance (112) entre la pupille d'entrée (304) et la cornée (306) étant ajoutée à la distance (110) entre l'œil (302) de la personne (300) et l'au moins un stimulus visuel (108) pour considérer la position de la pupille d'entrée (304).

11. Procédé de fabrication d'au moins un verre de lunettes, l'au moins un verre de lunettes étant fabriqué en traitant au moins une ébauche de verre en utilisant un modèle géométrique produit, le procédé comprenant la fabrication du modèle géométrique en réalisant le procédé de fabrication d'un modèle géométrique (500) d'au moins un verre de lunettes pour la fabrication de l'au moins un verre de lunettes selon la revendication précédente.

Fig. 1

200

Fig. 2

302

304

306

108

110

112

**Fig. 3**

404    404

400    402

406

**Fig. 4**

500    502

**Fig. 5**

## REFERENCES CITED IN THE DESCRIPTION

**Patent documents cited in the description**

- WO 2022090376 A1
- US 10702143 B2
- EP 3730038 A1

- WO 2021122640 A1
- US 20220313080 A1

**Non-patent literature cited in the description**

- **ROBERT E. BANNON**. A Study of Astigmatism at the near point with special reference to astigmatic accommodation. *American Journal of optometry and Archives of American academy of optometry*, February 1946, vol. 23 (2)
- **KENNETH N. OGLE** ; **J. THEODORE SCHWARTZ**. Depth of Focus of the Human Eye. *Journal of the optical society of America*, March 1959, vol. 49 (3)

- **ALEXANDER LEUBE** ; **CAROLINE KRAFT** ; **ARNE OHLENDORF** ; **SIEGFRIED WAHL**. Self-assessment of refractive errors using a simple optical approach. *Clinical and Experimental Optometry*, 2018, vol. 101 (3), 386-391
- **N. SHAH** ; **S. C. DAKIN** ; **T. REDMOND** ; **R. S. ANDERSON**. Vanishing Optotype Letters as a Clinical Acuity Test: Repeatability and Effect of the Number of Alternatives. *ARVO Annual Meeting Abstract*, April 2010
- **A.J. AUGUSTIN**. Augenheilkunde. Springer, 2007, 591